# EUROPEAN PATENT APPLICATION

(11) **EP 4 190 820 A1**
(43) Date of publication of application: **07.06.2023**
(21) Application number: 21849181.9
(22) Date of filing: 11.06.2021
(51) Int. Cl.: C07K 19/00, C12N 5/10, C12N 15/867, A61K 35/17, A61P 35/00

(54) **CHIMERIC ANTIGEN RECEPTOR AND USE THEREOF**

(30) Priority: 27.07.2020 CN 202010734872; 27.07.2020 CN 202010733636
(71) Applicant: Center for Excellence in Molecular Cell Science, Chinese Academy of Sciences, Shanghai 200031 (CN)
(72) Inventor: XU, Chenqi, Shanghai 200031 (CN); ZHOU, Qiuping, Shanghai 200031 (CN); WU, Wei, Shanghai 200031 (CN); XU, Xinyi, Shanghai 200031 (CN); HE, Xing, Shanghai 200031 (CN)
(74) Representative: Canzler & Bergmeier Patentanwälte Partnerschaft mbB
(86) International application number: PCT/CN2021/099798
(87) International publication number: WO 2022/022113

(57) **Abstract**

Provided is a chimeric antigen receptor containing a CD3ε intracellular region with a Y/F mutation. The structure of the chimeric antigen receptor comprises an extracellular domain, a transmembrane domain and an intracellular domain which are connected in sequence, wherein one end of the intracellular domain, which is connected to the transmembrane domain, is connected to the CD3ε intracellular region with the Y/F mutation, and the CD3ε intracellular region with the Y/F mutation refers to a Y/F mutant CD3ε intracellular region in which both tyrosines are mutated to phenylalanines in the ITAM of the CD3ε intracellular region. T cells modified with the chimeric antigen receptor have improved viability, decreased apoptosis level, and increased proliferation ability, and downregulate the levels of expression of cytokines IFN-γ and TNF-α.

## Description

### FIELD OF DISCLOSURE

The present disclosure relates to the field of chimeric antigen receptor, and in particular, to a chimeric antigen receptor containing a CD3ε intracellular region with a Y/F mutation or a chimeric antigen receptor containing a CD3ε intracellular basic residue rich sequence and use thereof.

### DESCRIPTION OF RELATED ARTS

The chimeric antigen receptor is referred to as CAR, and T cells carrying the CAR that targets a specific tumor antigen are referred to as CAR-T cells. CAR-T therapy has been widely applied to the treatment of tumors (especially immunotherapy of solid tumors) and is a novel and precise target therapy for tumors. CAR-T therapy has achieved a good effect in the clinical treatment of tumors through optimization and improvement in recent years. It is a promising novel immunotherapeutic approach that can accurately, quickly, and efficiently treat cancer, or even potentially cure cancer.

However, there are still many limitations to CAR-T therapy, for example, the problems during the clinical treatment of tumors include cytokine storms, neurotoxicity, and poor ability to continuous proliferation. The Lancet issued a phase I clinical trial (clinical trial number: NCT01593696, 2015.Lacct. T cells expressing CD19 chimeric antigen receptors for acute lymphoblastic leukaemia in children and young adults-a phase 1 dose-escalation trial) in 2015. The result showed that for the patients with relapsed or refractory acute lymphoblastic leukaemia or non-Hodgkin lymphoma (aged 1 - 30 years), after receiving KTE-C19 (anti-CD19 28Z) CAR-T therapy, the number of CAR-T cell in the blood peaked at day 14, followed by a significant decrease at day 28 and day 42 respectively, and no CAR-T cells were detected in the blood of all patients at day 68. In addition, The New England Journal of Medicine issued a phase I clinical trial (clinical trial number: NCT01044069, 2018.NEJ. Long-Term Follow-up of CD19 CAR Therapy in Acute Lymphoblastic Leukemia) in 2018. The result showed that the median of the number of CAR-T cells in the blood of 53 patients with relapsed acute lymphoblastic leukaemia appeared at day 14 after receiving anti-CD19 28Z CAR-T therapy. These two studies showed that 28Z CAR-T cells have a poor ability for continuous proliferation and viability, and the treatment efficacy needs to be improved.

### SUMMARY OF THE PRESENT INVENTION

An improvement in the CAR structure leads to an improvement in the continuous viability of CAR-T cells, thus improving the anti-tumor effect, prolonging the duration of disease remission, reducing the recurrence rate, and extending the survival period of patients. In view of the disadvantages of the prior art, the present disclosure provides a chimeric antigen receptor containing a CD3ε intracellular region with a Y/F mutation or CD3ε intracellular basic residue rich sequence and use thereof.

In order to achieve the above objectives and other related purposes, a first aspect of the present disclosure provides a chimeric antigen receptor containing a CD3ε intracellular region with a Y/F mutation, including:
an extracellular domain, a transmembrane domain, and an intracellular domain which are connected in sequence;
where the extracellular domain includes an antigen recognition region and a hinge region;
and one end of the intracellular domain which is connected to the transmembrane domain is connected to a CD3ε intracellular region with a Y/F mutation, where two tyrosines in the immunoreceptor tyrosine-based activation motif (ITAM) of the CD3ε intracellular region with a Y/F mutation are mutated into phenylalanine.

A second aspect of the present disclosure provides a polynucleotide sequence, which is selected from:
a polynucleotide sequence which encodes the aforementioned chimeric antigen receptor containing a CD3ε intracellular region with a Y/F mutation; and
(2) a complementary sequence of the polynucleotide sequence in (1).

A third aspect of the present disclosure provides a nucleic acid construct, including the polynucleotide sequence described in the second aspect of the present disclosure.

In an embodiment, the nucleic acid construct is a vector.

In an embodiment, the nucleic acid construct is a lentivirus vector containing a replication initiation site, a 3'LTR, a 5'LTR, and the polynucleotide sequence described in the second aspect of the present disclosure.

A fourth aspect of the present disclosure provides a lentivirus vector system, including the nucleic acid construct described in the third aspect of the present disclosure and a lentivirus vector auxiliary component.

A fifth aspect of the present disclosure provides a genetically modified T cell or a pharmaceutical composition including the genetically modified T cell, where the genetically modified T cell includes the polynucleotide sequence described in the second aspect of the present disclosure or nucleic acid construct described in the third aspect of the present disclosure, or infects the aforementioned lentivirus vector system described in the fourth aspect of the present disclosure.

A sixth aspect of the present disclosure provides a use of the chimeric antigen receptor containing a CD3ε intracellular region with a Y/F mutation described in the first aspect of the present disclosure, the polynucleotide sequence described in the second aspect of the present disclosure, the nucleic acid construct described in the third aspect of the present disclosure, or the lentivirus vector system described in the fourth aspect of the present disclosure in any one or more of the following: (1) preparing T cell; (2) improving T cell viability; (3) inhibiting T cells from apoptosis; (4) enhancing T cell proliferation and/or viability; (5) improving the anti-tumor effect of T cells; (6) inhibiting T cells from secreting cytokines IFN-γ and TNF-α.

A seventh aspect of the present disclosure provides a use of the chimeric antigen receptor containing a CD3ε intracellular region with a Y/F mutation described in the first aspect of the present disclosure, the polynucleotide sequence described in the second aspect of the present disclosure, nucleic acid construct described in the third aspect of the present disclosure, lentivirus vector system described in the fourth aspect of the present disclosure, or genetically modified T cells described in the fifth aspect of the present disclosure in the preparation of tumor treatment products.

As described above, the chimeric antigen receptor containing a CD3ε intracellular region with a Y/F mutation and the use thereof of the present disclosure have the following beneficial effects:

T cells modified with the chimeric antigen receptor containing a CD3ε intracellular region with a Y/F mutation herein improve the viability, decrease the apoptosis level, and increase the proliferation ability, so that T cell persistence is significantly improved, and the anti-tumor effect of T cells is significantly increased, which can further improve the therapeutic effect on the tumor, prolong the duration of disease remission, reduce the recurrence rate, and improve the survival period of patients in clinical application. At the same time, the chimeric antigen receptor-modified T cells can reduce the activation of macrophage and monocyte by down-regulating the expression of cytokines IFN-γ and TNF-α to decrease the amount of inflammatory cytokines such as IL-1β and IL-6.

In order to achieve the above objectives and other related purposes, an eighth aspect of the present disclosure provides a chimeric antigen receptor containing a CD3ε intracellular basic residue rich sequence, including:
an extracellular domain, a transmembrane domain, and an intracellular domain which are connected in sequence;
where the extracellular domain includes an antigen recognition region and a hinge region;
and one end of the intracellular domain which is connected to the transmembrane domain is connected to a CD3ε intracellular basic residue rich sequence.

A ninth aspect of the present disclosure provides a polynucleotide sequence, which is selected from:
a polynucleotide sequence which encodes the chimeric antigen receptor containing a CD3ε intracellular basic residue rich sequence described in the eighth aspect of the present disclosure; and
(2) a complementary sequence of the polynucleotide sequence in (1).

A tenth aspect of the present disclosure provides a nucleic acid construct, including the polynucleotide sequence described in the ninth aspect of the present disclosure.

In an embodiment, the nucleic acid construct is a vector.

In an embodiment, the nucleic acid construct is a lentivirus vector containing a replication initiation site, a 3'LTR, a 5'LTR, and the polynucleotide sequence described in the ninth aspect of the present disclosure.

An eleventh aspect of the present disclosure provides a lentivirus vector system, including the nucleic acid construct described in the tenth aspect of the present disclosure and a lentivirus vector auxiliary component.

A twelfth aspect of the present disclosure provides a genetically modified T cell or a pharmaceutical composition including the genetically modified T cell, where the genetically modified T cell includes the polynucleotide sequence described in the ninth aspect of the present disclosure or the nucleic acid construct described in the tenth aspect of the present disclosure, or infects the lentivirus vector system described in the eleventh aspect.

A thirteenth aspect of the present disclosure provides a use of the chimeric antigen receptor containing a CD3ε intracellular basic residue rich sequence described in the eighth aspect of the present disclosure, the polynucleotide sequence described in the ninth aspect of the present disclosure, the nucleic acid construct described in the tenth aspect of the present disclosure, or the lentivirus vector system described in the eleventh aspect of the present disclosure in any one or more of the following: (1) preparing T cells; (2) improving T cell viability; (3) inhibiting T cells form apoptosis; (4) enhancing T cell proliferation; (5) improving the anti-tumor effect of T cells; (6) inhibiting T cells from secreting cytokine IFN-γ.

A fourteenth aspect of the present disclosure provides a use of the chimeric antigen receptor containing a CD3ε intracellular basic residue rich sequence described in the eighth aspect of the present disclosure, the polynucleotide sequence described in the ninth aspect of the present disclosure, the nucleic acid construct described in the tenth aspect of the present disclosure, or the lentivirus vector system described in the eleventh aspect of the present disclosure, or the genetically modified T cells described in the twelfth aspect of the present disclosure in the preparation of tumor treatment products.

As described above, the chimeric antigen receptor containing a CD3ε intracellular basic residue rich sequence and the use thereof of the present disclosure have the following beneficial effects:

T cells modified with the chimeric antigen receptor containing a CD3ε intracellular basic residue rich sequence herein improve the viability, decrease the apoptosis level, and increase the proliferation ability, so that T cell persistence is significantly improved, and the anti-tumor effect of T cells is significantly increased, which can further improve the therapeutic effect on the tumor, prolong the duration of disease remission, reduce the recurrence rate, and improve the survival period of patients in clinical application. At the same time, the chimeric antigen receptor-modified T cells can reduce the activation of macrophage and monocyte by down-regulating the expression of cytokines IFN-γ to decrease the amount of inflammatory cytokines such as IL-1β and IL-6.

### BRIEF DESCRIPTION OF THE DRAWINGS

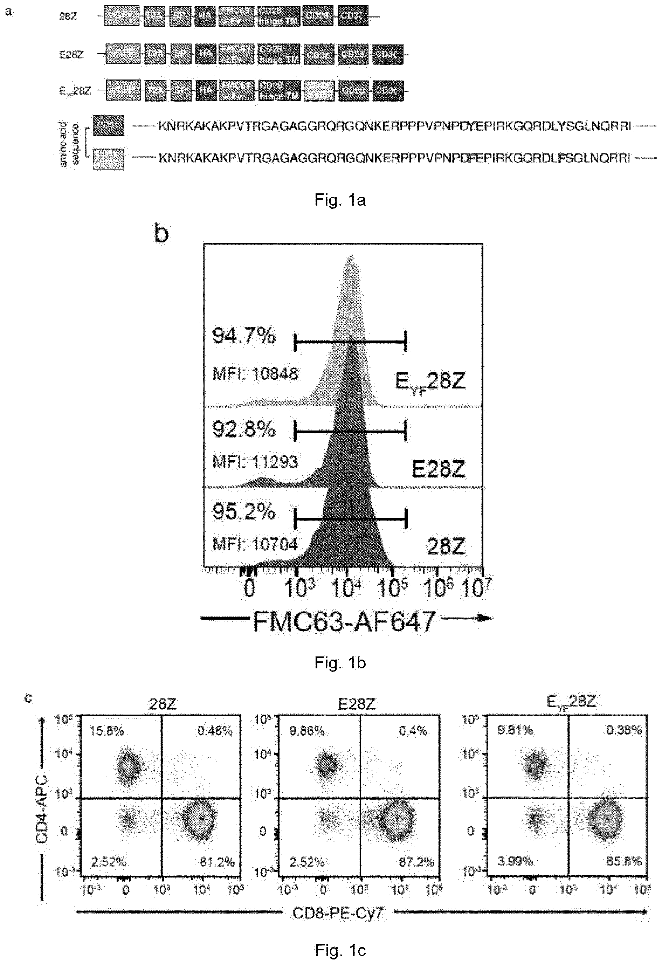

Fig. 1a. shows a structural schematic diagram of anti-CD19 28Z CAR, anti-CD19 E28Z CAR, and anti-CD19 E_{YF}28Z CAR, and shows amino acid sequences of CD3ε intracellular region and CD3ε intracellular region with a Y/F mutation, where FMC63 is a single-chain antibody targeting the CD19 antigen, and the hinge region and transmembrane region of the receptor derive from human CD28;
in anti-CD19 E28Z CAR, CD3ε is inserted behind the CD28 transmembrane region and before the CD28 intracellular region, in anti-CD19 E_{YF}28Z CAR, CD3ε intracellular region with a Y/F mutation is inserted behind the CD28 transmembrane region and before the CD28 intracellular region.
Fig. 1b. shows a flow diagram of anti-CD19 28Z CAR, anti-CD19 E28Z CAR, and anti-CD19 E_{YF}28Z CAR on membrane surfaces after T cell expression.
Fig. 1c. shows a flow diagram of CD4⁺ / CD8⁺ cell ratios in anti-CD19 28Z CAR-T cells, anti-CD19 E28Z CAR-T cells, and anti-CD19 E_{YF}28Z CAR-T cells.

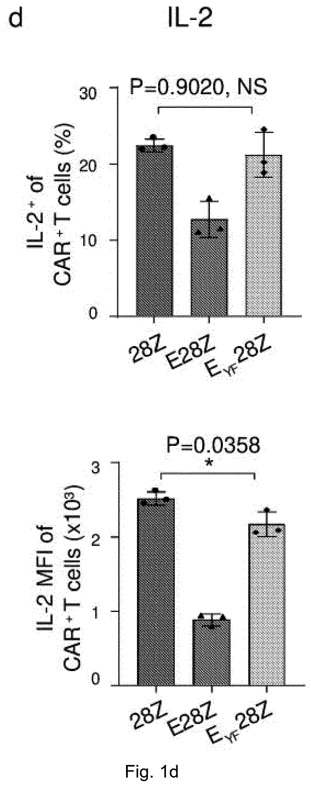

Fig. 1d. shows IL-2 cytokine levels of anti-CD19 28Z CAR-T cells, anti-CD19 E28Z CAR-T cells, and anti-CD19 E_{YF}28Z CAR-T cells after Raji cell (CD19 antigen) stimulation.

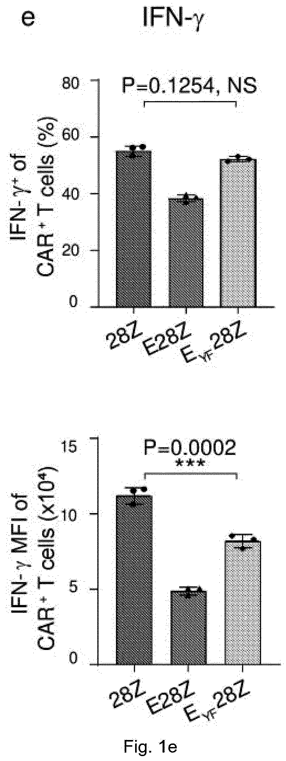

Fig. 1e. shows IFN-γ cytokine levels of anti-CD19 28Z CAR-T cells, anti-CD19 E28Z CAR-T cells, and anti-CD19 E_{YF}28Z CAR-T cells after Raji cell (CD19 antigen) stimulation.

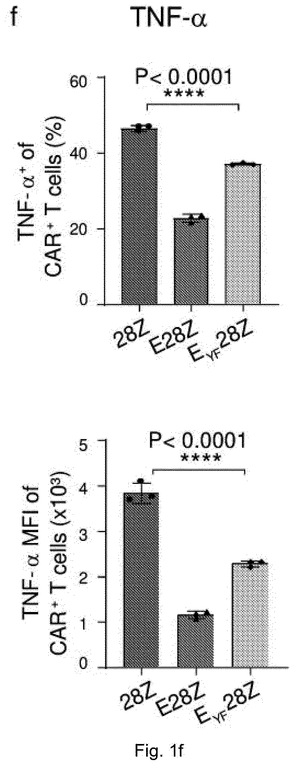

Fig. 1f. shows TNF-α cytokine levels of anti-CD19 28Z CAR-T cells, anti-CD19 E28Z CAR-T cells, and anti-CD19 E_{YF}28Z CAR-T cells after Raji cell (CD19 antigen) stimulation.

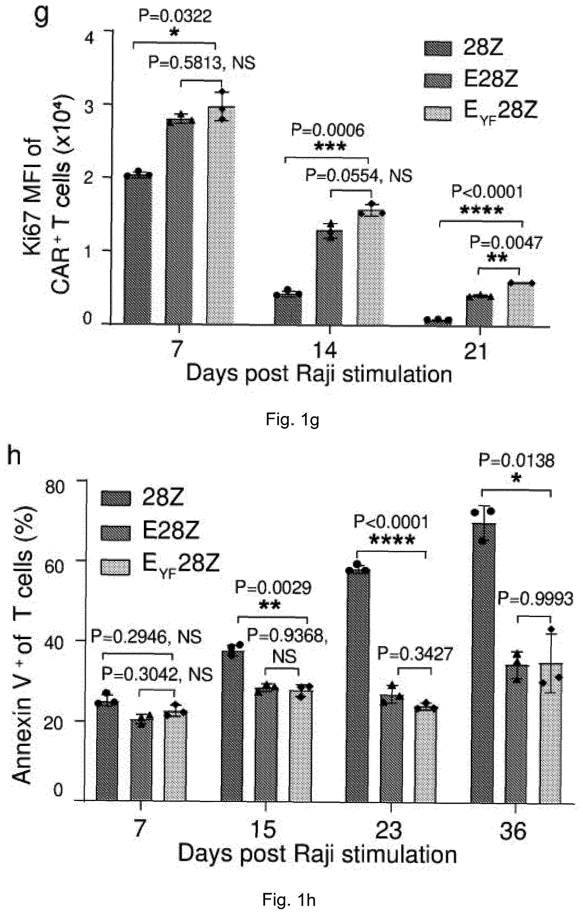

Fig. 1g. shows proliferation capacities of anti-CD19 28Z CAR-T cells, anti-CD19 E28Z CAR-T cells, and anti-CD19 E_{YF}28Z CAR-T cells after Raji cell (CD19 antigen) stimulation.
Fig. 1h. shows apoptosis levels of anti-CD19 28Z CAR-T cells, anti-CD19 E28Z CAR-T cells, and anti-CD19 E_{YF}28Z CAR-T cells after Raji cell (CD19 antigen) stimulation.
Fig. 1 i. shows survival levels of anti-CD19 28Z CAR-T cells, anti-CD19 E28Z CAR-T cells, and anti-CD19 E_{YF}28Z CAR-T cells after Raji cell (CD19 antigen) stimulation.
Fig. 1j. shows cytotoxicity levels of anti-CD19 28Z CAR-T cells, anti-CD19 E28Z CAR-T cells, and anti-CD19 E_{YF}28Z CAR-T cells against CD19⁺ tumor cells.
Fig. 1k. shows the levels of phosphorylation (pErk(1/2)^{Thr202/Try204}) of the signaling molecule serine/threonine protein kinase Erk(1/2)^{Thr202/Tyr204} that promotes the proliferation, viability, and anti-apoptosis of anti-CD19 28Z CAR-T cells, anti-CD19 E28Z CAR-T cells, and anti-CD19 E_{YF}28Z CAR-T cells, after Raji cell (CD19 antigen) stimulation.
Fig. 1l. shows the levels of phosphorylation (pAKT^{S473}) of the signaling molecule serine/threonine kinase AKT^{S473} that promotes the proliferation, viability, and anti-apoptosis of anti-CD19 28Z CAR-T cells, anti-CD19 E28Z CAR-T cells, and anti-CD19 E_{YF}28Z CAR-T cells, after Raji cell (CD19 antigen) stimulation.
Fig. 1m. shows the levels of phosphorylation (pS6^{S235/236}) of the signaling molecule ribosomal protein S6^{S235/236} that indicates the growth and proliferation of anti-CD19 28Z CAR-T cells, anti-CD19 E28Z CAR-T cells, and anti-CD19 E_{YF}28Z CAR-T cells, after Raji cell (CD19 antigen) stimulation.
Fig. 2a. shows a structural schematic diagram of anti-CD19 28Z CAR and anti-CD19 E_{BRS}28Z CAR, where FMC63 is a single-chain antibody targeting CD19 antigen, the hinge region and transmembrane region of the receptor derive from human CD28, and a CD3ε intracellular basic residue rich sequence is inserted behind the CD28 transmembrane region and before the CD28 intracellular region.
Fig. 2b. shows a flow diagram of anti-CD19 28Z CAR and anti-CD19 E_{BRS}28Z CAR on membrane surfaces after T cell expression.
Fig. 2c. shows a flow diagram of CD4⁺ / CD8⁺ cell ratios of anti-CD19 28Z CAR-T cells and anti-CD19 E_{BRS}28Z CAR-T cells.
Figs. 2d. shows IL-2 cytokine levels of anti-CD19 28Z CAR-T cells and anti-CD19 E_{BRS}28Z CAR-T cells after Raji cell (CD19 antigen) stimulation.
Figs. 2e. shows IFN-γ cytokine levels of anti-CD19 28Z CAR-T cells and anti-CD19 E_{BRS}28Z CAR-T cells after Raji cell (CD19 antigen) stimulation.
Figs. 2f. shows TNF-α cytokine levels of anti-CD19 28Z CAR-T cells and anti-CD19 E_{BRS}28Z CAR-T cells after Raji cell (CD19 antigen) stimulation.
Fig. 2g. shows proliferation capacities of anti-CD19 28Z CAR-T cells and anti-CD19 E_{BRS}28Z CAR-T cells after Raji cell (CD19 antigen) stimulation.
Fig. 2h. shows apoptosis levels of anti-CD19 28Z CAR-T cells and anti-CD19 E_{BRS}28Z CAR-T cells after Raji cell (CD19 antigen) stimulation.
Fig. 2i. shows survival levels of anti-CD19 28Z CAR-T cells and anti-CD19 E_{BRS}28Z CAR-T cells after Raji cell (CD19 antigen) stimulation.
Fig. 2j. shows phosphorylation levels of the signaling molecule serine/threonine kinase AKT^{S473} that promotes the proliferation, viability, and anti-apoptosis of anti-CD19 28Z CAR-T cells and anti-CD19 E_{BRS}28Z CAR-T cells after Raji cell (CD19 antigen) stimulation.
Fig. 2k. shows phosphorylation levels of the signaling molecule ribosomal protein S6^{S235/236} that indicates the growth and proliferation of anti-CD19 28Z CAR-T cells and anti-CD19 E_{BRS}28Z CAR-T cells after Raji cell (CD19 antigen) stimulation.
Fig. 2l. shows cytotoxicity levels of anti-CD19 28Z CAR-T cells and anti-CD19 E_{BRS}28Z CAR-T cells against CD19⁺ tumor cells.

### DETAILED DESCRIPTION OF THE PREFERRED EMBODIMENTS

The chimeric antigen receptor containing a CD3ε intracellular region with a Y/F mutation of the present disclosure, includes:
an extracellular domain, a transmembrane domain, and an intracellular domain which are connected in sequence;
where the extracellular domain contains an antigen recognition region and a hinge region;
and one end of the intracellular domain which is connected to the transmembrane domain is connected to a CD3ε intracellular region with a Y/F mutation, where the CD3ε intracellular region with a Y/F mutation refers to the CD3ε intracellular region in which two tyrosines in the immunoreceptor tyrosine-based activation motif (ITAM) are mutated into phenylalanine.

Further, the amino acid sequence of the CD3ε intracellular region with a Y/F mutation is shown as SEQ ID NO: 1, specifically:

KNRKAKAKPVTRGAGAGGRQRGQNKERPPPVPNPDFEPIRKGQRDLFSG
LNQRRI.

The intracellular domain includes the CD3ε intracellular region with a Y/F mutation (two tyrosines in the ITAM are mutated into phenylalanine), a costimulatory signaling region, and a CD3ζ intracellular segment which are connected in sequence.

In an embodiment, the costimulatory signaling region is selected from one or more of intracellular segments of CD27, CD28, CD134, 4-1BB, OX40, and ICOS.

In a further embodiment, the costimulatory signaling region is selected from the CD28 intracellular segment, whose killing capability is stronger.

The amino acid sequence of the CD28 intracellular segment is shown as SEQ ID NO: 2, specifically:
RSKRSRLLHSDYMNMTPRRPGPTRKHYQPYAPPRDFAAYRS.

In an embodiment, the amino acid sequence of the CD3ζ intracellular segment is shown as SEQ ID NO: 3, specifically:

RVKFSRSADAPAYQQGQNQLYNELNLGRREEYDVLDKRRGRDPEMGGKP
RRKNPQEGLYNELQKDKMAEAYSEIGMKGERRRGKGHDGLYQGLSTATKD
TYDALHMQALPPR.

In an embodiment, the antigen recognition region is a single-chain antibody against a tumor surface antigen, where the tumor surface antigen is selected from one or more of CD19, mesothelin, CD20, CD22, CD123, CD30, CD33, CD38, CD138, BCMA, Fibroblast activation protein (FAP), Glypican-3, CEA, EGFRvIII, PSMA, Her2, IL13Rα2, CD171, and GD2.

In an embodiment, the tumor surface antigen is selected from CD19 and mesothelin, which results in good targeting specificity.

The transmembrane domain is selected from one or more of transmembrane regions of CD28, CD4, CD8α, OX40, and H2-Kb.

In an embodiment, the transmembrane domain is selected from the CD28 transmembrane region. The amino acid sequence of the CD28 transmembrane region is shown as SEQ ID NO: 4, specifically:
FWVLWVGGVLACYSLLVTVAFIIFWV.

The hinge region is selected from one or more of CD28 hinge region, CD8α hinge region, CD4 hinge region, IgG hinge region, or a coupled hinge region of IgG hinge region and CH2CH3 region, that is, the hinge region may be IgG1 hinge or IgG1 hinge-CH2CH3.

In an embodiment, the hinge region is the CD28 hinge region. The corresponding amino acid structure is shown as SEQ ID NO: 7, specifically:
IEVMYPPPYLDNEKSNGTIIHVKGKHLCPSPLFPGPSKP.

In an embodiment, the single-chain antibody includes a light chain variable region and a heavy chain variable region.

In an embodiment, the light chain variable region and the heavy chain variable region are connected by a linker sequence.

In an embodiment, the single-chain antibody is selected from FMC63.

FMC63 may be a commercially available product.

In an embodiment, the single-chain antibody includes a light chain variable region and a heavy chain variable region of a monoclonal antibody FMC63, and the light chain variable region and the heavy chain variable region are optionally connected by a linker.

In one embodiment, the antigen recognition region of the chimeric antigen receptor containing a CD3ε intracellular region with a Y/F mutation is selected from FMC63, the hinge region is selected from the CD28 hinge region, the transmembrane domain is selected from the CD28 transmembrane region, and the intracellular domain includes the CD3ε intracellular region with a Y/F mutation, a CD28 intracellular segment, and a CD3ζ intracellular segment which are connected in sequence.

In one embodiment, the amino acid sequence of the chimeric antigen receptor containing a CD3ε intracellular region with a Y/F mutation (Anti-CD19 E_{YF}28Z CAR) is shown as SEQ ID NO: 5, specifically:

SEQ ID NO: 5 (the amino acid sequence of Anti-CD19 E_{YF}28Z CAR): (5'→3') starting from FMC63 scFv, then CD28 hinge region, CD28 transmembrane region, CD3ε intracellular region with a Y/F mutation, and CD3ζ intracellular region are connected sequentially:

DIQMTQTTSSLSASLGDRVTISCRASQDISKYLNWYQQKPDGTVKL
LIYHTSRLHSGVPSRFSGSGSGTDYSLTISNLEQEDIATYFCQQGNTLPYTF
GGGTKLEITGSTSGSGKPGSGEGSTKGEVKLQESGPGLVAPSQSLSVTCT
VSGVSLPDYGVSWIRQPPRKGLEWLGVIWGSETTYYNSALKSRLTIIKDNSK
SQVFLKMNSLQTDDTAIYYCAKHYYYGGSYAMDYWGQGTSVTVSSAAAIE
VMYPPPYLDNEKSNGTIIHVKGKHLCPSPLFPGPSKPFWVLVVVGGVLACY
SLLVTVAFIIFWVKNRKAKAKPVTRGAGAGGRQRGQNKERPPPVPNPDFEP
IRKGQRDLFSGLNQRRIRSKRSRLLHSDYMNMTPRRPGPTRKHYQPYAPP

RDFAAYRSRVKFSRSADAPAYQQGQNQLYNELNLGRREEYDVLDKRRGRD
PEMGGKPRRKNPQEGLYNELQKDKMAEAYSEIGMKGERRRGKGHDGLYQ
GLSTATKDTYDALHMQALPPR.

The nucleotide sequence corresponding to SEQ ID NO: 5 (the amino acid sequence of Anti-CD19 E_{YF}28Z CAR) is shown as SEQ ID NO: 6 (the nucleotide sequence of Anti-CD19 E_{YF}28Z CAR). SEQ ID NO: 6 is as follows:

(5'→3')
gacatccagatgacacagactacatcctccctgtctgcctctctgggagacagagtcaccatcagttgcag
ggcaagtcaggacattagtaaatatttaaattggtatcagcagaaaccagatggaactgttaaactcctgat
ctaccatacatcaagattacactcaggagtcccatcaaggttcagtggcagtgggtctggaacagattattc
tctcaccattagcaacctggagcaagaagatattgccacttacttttgccaacagggtaatacgcttccgtac
acgttcggaggggggactaagttggaaataacaggctccacctctggatccggcaagcccggatctggc
gagggatccaccaagggcgaggtgaaactgcaggagtcaggacctggcctggtggcgccctcacaga
gcctgtccgtcacatgcactgtctcaggggtctcattacccgactatggtgtaagctggattcgccagcctcc
acgaaagggtctggagtggctgggagtaatatggggtagtgaaaccacatactataattcagctctcaaat
ccagactgaccatcatcaaggacaactccaagagccaagttttcttaaaaatgaacagtctgcaaactga
tgacacagccatttactactgtgccaaacattattactacggtggtagctatgctatggactactggggtcaa
ggaacctcagtcaccgtctcctcagcggccgcaattgaagttatgtatcctcctccttacctagacaatgag
aagagcaatggaaccattatccatgtgaaagggaaacacctttgtccaagtcccctatttcccggaccttct
aagcccttttgggtgctggtggtggttgggggagtcctggcttgctatagcttgctagtaacagtggcctttatt
attttctgggtgaagaatagaaaggccaaggccaagcctgtgacacgaggagcgggtgctggcggcag
gcaaaggggacaaaacaaggagaggccaccacCTGTTCCCAACCCAGACTTTGAGC
CCATCCGGAAAGGCCAGCGGGACCTGTTTTCTGgcctgaatcagagacgcatca
ggagtaagaggagcaggctcctgcacagtgactacatgaacatgactccccgccgccccgggcccacc
cgcaagcattaccagccctatgccccaccacgcgacttcgcagcctatcgctccagagtgaagttcagca
ggagcgcagacgcccccgcgtaccagcagggccagaaccagctctataacgagctcaatctaggacg
aagagaggagtacgatgttttggacaagagacgtggccgggaccctgagatggggggaaagccgaga
aggaagaaccctcaggaaggcctgtacaatgaactgcagaaagataagatggcggaggcctacagtg
agattgggatgaaaggcgagcgccggaggggcaaggggcacgatggcctttaccagggtctcagtaca
gccaccaaggacacctacgacgcccttcacatgcaggccctgccccctcgctaa.

The chimeric antigen receptor containing a CD3ε intracellular basic residue rich sequence of the present disclosure, includes:
an extracellular domain, a transmembrane domain, and an intracellular domain which are connected in sequence;
where the extracellular domain contains an antigen recognition region and a hinge region;
and one end of the intracellular domain which is connected to the transmembrane domain is connected to a CD3ε intracellular basic residue rich sequence (BRS).

Further, the amino acid sequence of the CD3ε intracellular basic residue rich sequence is shown as SEQ ID NO: 10, specifically: KNRKAKAK.

The intracellular domain includes the CD3ε intracellular basic residue rich sequence, a costimulatory signaling region, and a CD3ζ intracellular segment that are sequentially connected.

In an embodiment, the costimulatory signaling region is selected from one or more of intracellular segments of CD27, CD28, CD134, 4-1BB, OX40, and ICOS.

In a further embodiment, the costimulatory signaling region is selected from CD28 intracellular segment, whose killing capability is stronger.

The amino acid sequence of CD28 intracellular segment is shown as SEQ ID NO: 2; specifically:
RSKRSRLLHSDYMNMTPRRPGPTRKHYQPYAPPRDFAAYRS.

In an embodiment, the amino acid sequence of the CD3ζ intracellular segment is shown as SEQ ID NO: 3, specifically:

RVKFSRSADAPAYQQGQNQLYNELNLGRREEYDVLDKRRGRDPEMGGKP
RRKNPQEGLYNELQKDKMAEAYSEIGMKGERRRGKGHDGLYQGLSTATKD
TYDALHMQALPPR.

In an embodiment, the antigen recognition region is a single-chain antibody against a tumor surface antigen, where the tumor surface antigen is selected from one or more of CD19, mesothelin, CD20, CD22, CD123, CD30, CD33, CD38, CD138, BCMA, Fibroblast activation protein (FAP), Glypican-3, CEA, EGFRvIII, PSMA, Her2, IL13Rα2, CD171, and GD2.

Further, the tumor surface antigen is selected from CD19 and mesothelin, which results in good targeting specificity.

Further, the transmembrane domain is selected from one or more of transmembrane regions of CD28, CD4, CD8α, OX40, and H2-Kb.

In an embodiment, the transmembrane domain is selected from the CD28 transmembrane region. The amino acid sequence of the CD28 transmembrane region is shown as SEQ ID NO: 4, specifically,
FWVLWVGGVLACYSLLVTVAFIIFWV.

The hinge region is selected from one or more of CD28 hinge region, CD8α hinge region, CD4 hinge region, IgG hinge region, or a coupled hinge region of IgG hinge region and CH2CH3 region, that is, the hinge region may be IgG1 hinge or IgG1 hinge-CH2CH3.

In an embodiment, the hinge region is CD28 hinge region. The corresponding amino acid structure is shown as SEQ ID NO: 7, specifically,
IEVMYPPPYLDNEKSNGTIIHVKGKHLCPSPLFPGPSKP.

In an embodiment, the single-chain antibody includes a light chain variable region and a heavy chain variable region.

In an embodiment, the light chain variable region and the heavy chain variable region are connected by a linker sequence.

In an embodiment, the single-chain antibody is selected from FMC63.

FMC63 may be a commercially available product.

In an embodiment, the single-chain antibody includes a light chain variable region and a heavy chain variable region of a monoclonal antibody FMC63, and the light chain variable region and the heavy chain variable region are optionally connected by a linker.

The antigen recognition region of the chimeric antigen receptor containing a CD3ε intracellular basic residue rich sequence is selected from FMC63, the hinge region is selected from CD28 hinge region, the transmembrane domain is selected from the CD28 transmembrane region, the intracellular domain includes the CD3ε intracellular basic residue rich sequence, a CD28 intracellular segment, and a CD3ζ intracellular segment which are sequentially connected.

In one embodiment, the chimeric antigen receptor containing a CD3ε intracellular basic residue rich sequence (Anti-CD19 E_{BRS}28Z CAR) includes: FMC63 scFv, CD28 hinge region, CD28 transmembrane region, CD3ε BRS, and CD3ζ intracellular region.

In one embodiment, the amino acid sequence of the chimeric antigen receptor containing a CD3ε intracellular basic residue rich sequence (Anti-CD19 E_{BRS}28Z CAR) is shown as SEQ ID NO: 11, specifically:

SEQ ID NO: 11 (the amino acid sequence of Anti-CD19 E_{BRS}28Z CAR): (5'→3') starting from FMC63 scFv, then CD28 hinge region, CD28 transmembrane region, CD3ε BRS, and CD3ζ intracellular region are connected sequentially:

DIQMTQTTSSLSASLGDRVTISCRASQDISKYLNWYQQKPDGTVKL
LIYHTSRLHSGVPSRFSGSGSGTDYSLTISNLEQEDIATYFCQQGNTLPYTF
GGGTKLEITGSTSGSGKPGSGEGSTKGEVKLQESGPGLVAPSQSLSVTCT
VSGVSLPDYGVSWIRQPPRKGLEWLGVIWGSETTYYNSALKSRLTIIKDNSK
SQVFLKMNSLQTDDTAIYYCAKHYYYGGSYAMDYWGQGTSVTVSSAAAIE
VMYPPPYLDNEKSNGTIIHVKGKHLCPSPLFPGPSKPFWVLVVVGGVLACY
SLLVTVAFIIFWVKNRKAKAKRSKRSRLLHSDYMNMTPRRPGPTRKHYQPY
APPRDFAAYRSRVKFSRSADAPAYQQGQNQLYNELNLGRREEYDVLDKRR
GRDPEMGGKPRRKNPQEGLYNELQKDKMAEAYSEIGMKGERRRGKGHD
GLYQGLSTATKDTYDALHMQALPPR.

**The** nucleotide sequence corresponding to the amino acid sequence of Anti-CD19 E_{BRS}28Z CAR is shown as SEQ ID NO: 12 (the nucleotide sequence of Anti-CD19 E_{BRS}28Z CAR). SEQ ID NO: 12 is as follows: (5'→3')

gacatccagatgacacagactacatcctccctgtctgcctctctgggagacagagtcaccatc
agttgcagggcaagtcaggacattagtaaatatttaaattggtatcagcagaaaccagatggaactgttaa
actcctgatctaccatacatcaagattacactcaggagtcccatcaaggttcagtggcagtgggtctggaac
agattattctctcaccattagcaacctggagcaagaagatattgccacttacttttgccaacagggtaatacg
cttccgtacacgttcggaggggggactaagttggaaataacaggctccacctctggatccggcaagcccg
gatctggcgagggatccaccaagggcgaggtgaaactgcaggagtcaggacctggcctggtggcgcc
ctcacagagcctgtccgtcacatgcactgtctcaggggtctcattacccgactatggtgtaagctggattcgc
cagcctccacgaaagggtctggagtggctgggagtaatatggggtagtgaaaccacatactataattcag
ctctcaaatccagactgaccatcatcaaggacaactccaagagccaagttttcttaaaaatgaacagtctg
caaactgatgacacagccatttactactgtgccaaacattattactacggtggtagctatgctatggactact

ggggtcaaggaacctcagtcaccgtctcctcagcggccgcaattgaagttatgtatcctccttacctag
acaatgagaagagcaatggaaccattatccatgtgaaagggaaacacctttgtccaagtcccctatttccc
ggaccttctaagcccttttgggtgctggtggtggttgggggagtcctggcttgctatagcttgctagtaacagtg
gcctttattattttctgggtgaagaatagaaaggccaaggccaagaggagtaagaggagcaggctcctgc
acagtgactacatgaacatgactccccgccgccccgggcccacccgcaagcattaccagccctatgccc
caccacgcgacttcgcagcctatcgctccagagtgaagttcagcaggagcgcagacgcccccgcgtac
cagcagggccagaaccagctctataacgagctcaatctaggacgaagagaggagtacgatgttttggac
aagagacgtggccgggaccctgagatggggggaaagccgagaaggaagaaccctcaggaaggcct
gtacaatgaactgcagaaagataagatggcggaggcctacagtgagattgggatgaaaggcgagcgc
cggaggggcaaggggcacgatggcctttaccagggtctcagtacagccaccaaggacacctacgacg
cccttcacatgcaggccctgccccctcgctaa.

The above-mentioned parts of the chimeric antigen receptor in the present disclosure may be connected directly with each other or connected through the linker sequence. The linker sequence may be any linker sequence suitable for antibody and known in this field, for example, the linker sequence may contain G and S. Generally speaking, the linker may contain one or more repeated motifs. For example, the motif may be GGGS, GGGGS, SSSSG, GSGSA, and GGSGG. In an embodiment, the motifs of the linker sequence are adjacent to each other and no amino acid residue is inserted in the repeated motifs. The linker sequence may include 1, 2, 3, 4, or 5 repeated motifs, and the length of the linker may be 3-25 amino acid residues, such as 3-15, 5-15, or 10-20 amino acid residues. In some embodiments, the linker sequence may contain multiple glycines. The number of glycine in the linker sequence is not particularly limited, and may be 2-20, for example, 2-15, 2-10, or 2-8. Except for glycine and serine, the linker may also contain other known amino acid residues, such as alanine (A), leucine (L), threonine (T), glutamic acid (E), phenylalanine (F), arginine (R), and glutamine (Q).

It should be understood that a restriction enzyme site is usually needed in gene cloning, therefore, one or more unrelated residues will be introduced to a terminal of the amino acid sequence to be expressed, however, this operation has no effect on the activity of the targeted sequence. In order to construct a fusion protein, promote the expression of a recombinant protein, obtain the recombinant protein that is secreted out of the host automatically, or purify the recombinant protein, it is usually required to add some amino acids to an N-terminal, a C-terminal, or other proper regions in the recombinant protein, such as, including but not limited to, proper adaptor peptide, signal peptide, leader peptide, etc. According to the signal peptide hypothesis, the mRNA encoding secreted protein first synthesizes a signal peptide with hydrophobic amino acid residues at the N-terminal during translation, and the signal peptide is then recognized and combined with the receptor on the endoplasmic reticulum membrane. The signal peptide reaches the lumen of the endoplasmic reticulum via the pore formed by the protein in the membrane, and then is hydrolyzed by the signal peptidase located on the surface of the lumen, therefore, the new polypeptide can enter the lumen of the endoplasmic reticulum and finally be secreted to the extracellular according to the above guidance. The signal peptides related to CAR have different sources, mainly CD8 and CD28, and GM-CSF receptor signal peptides. The amino terminal or carboxyl terminal of the fusion protein (i.e. the CAR) of the present disclosure may include one or more polypeptide fragments to serve as protein tags. Any suitable tag may be used herein. For example, the tag may be FLAG, HA, HA1, c-Myc, Poly-His, Poly-Arg, Strep-Tagll, AU1, EE, T7, 4A6, ε, B, gE, and Ty1. These tags may be used for protein purification.

**The** polynucleotide sequence provided by the present disclosure is selected from:
**(1)** a polynucleotide sequence encoding the aforementioned chimeric antigen receptor; and
(2) a complementary sequence to the polynucleotide sequence in (1).

**The** polynucleotide sequence of the present disclosure may be in the form of DNA or RNA. DNA includes cDNA, genomic DNA, or synthetic DNA. The DNA may be single-stranded or double-stranded. The DNA may be a coding strand or a non-coding strand. The present disclosure also includes a variant of the polynucleotide sequence which encodes the fusion protein, i.e., a nucleotide sequence encoding the same amino acid sequence but having a different nucleotide sequence.

The polynucleotide sequences described herein may be obtained by PCR amplification. Specifically, primers can be designed according to the nucleotide sequences disclosed herein, in particular, according to open reading frame sequences, and related sequences can be obtained by using a commercially available cDNA library or a cDNA library prepared according to conventional methods known to a person skilled in the art as a template. When the sequence is relatively long, two or more PCR amplifications are needed, and then the amplified fragments are spliced together in the correct order.

**The** nucleic acid construct provided by the present disclosure includes the above polynucleotide sequence.

The nucleic acid construct further includes one or more regulatory sequences operatively connected to the aforementioned polynucleotide sequence. The coding sequence of the CAR of the present disclosure can be operated in a variety of ways to ensure the expression of the protein. The nucleic acid construct may be operated according to different expression vectors or requirements of the expression vector before inserting the nucleic acid construct into the vector. Altering polynucleotide sequences by using the recombinant DNA method is known in the art.

The regulatory sequence may be a suitable promoter sequence. The promoter sequence is usually operatively connected to the coding sequence of the protein to be expressed. The promoter may be any nucleotide sequence that shows transcriptional activity in the selected host cell, including mutated, truncated, and hybrid promoters, and may be obtained from genes encoding extracellular or intracellular polypeptides which are homologous or heterologous to the host cell.

The regulatory sequence may also be a suitable transcription terminator sequence, i.e., the sequence identified by the host cell to terminate the transcription. The terminator sequence is operatively connected to the 3' terminal of the nucleotide sequence encoding the polypeptide. Any terminator with functionality in the selected host cell may be used in the present disclosure.

The regulatory sequence may also be a suitable leader sequence, i.e., an untranslated region for mRNA which is important to the translation of a host cell. The leader sequence is operably linked to the 5' terminal of the nucleotide sequence encoding the polypeptide. Any leader sequence with functionality in the selected host cell may be used in the present disclosure.

In an embodiment, the nucleic acid construct is a vector.

The expression of the polynucleotide sequence encoding the CAR is typically achieved by operably linking the polynucleotide sequence encoding the CAR to the promoter and incorporating the construct into the expression vector. The vector may be suitable for the replication and integration of eukaryotic cells. Typical cloning vectors include transcription and translation terminators, initiating sequences, and promoters that can be used for modulating the expression of desired nucleic acid sequences.

The polynucleotide sequence encoding the CAR of the present disclosure may be cloned into many types of vectors, for example, plasmid, phagemid, phage derivative, animal virus, and cosmid. Further, the vector is an expression vector. The expression vector may be provided to the cell in the form of a virus vector. Virus vector technology is known in the art. Viruses that can be used as vectors include but are not limited to, retroviruses, adenoviruses, adeno-associated viruses, herpesviruses, and lentivirus. Generally, a suitable vector includes a replication origin, a promoter sequence, a convenient restriction enzyme site, and one or more selectable tags that function in at least one organism.

In an embodiment, the nucleic acid construct is a lentivirus vector containing the replication origin, 3'LTR, 5'LTR, and the foregoing polynucleotide sequence.

One example of a suitable promoter is an immediate early cytomegalovirus (CMV) promoter sequence. The promoter sequence is a strong constitutive promoter sequence capable of driving high-level expression of any polynucleotide sequence operably linked thereto. Another example of a suitable promoter is an elongation factor-1α (EF-1α). However, other constitutive promoter sequences may also be used, including but not limited to, simian virus 40 (SV40) early promoter, mouse mammary tumor virus (MMTV), human immunodeficiency virus (HIV) long terminal repeated (LTR) promoter, MoMuLV promoter, avian leukosis virus promoter, EB virus immediate early promoter, Rous sarcoma virus promoter, and human gene promoter (including but not limited to actin promoter, myosin promoter, heme promoter, and creatine kinase promoter). Further, the use of an inducible promoter may also be considered. The use of an inducible promoter provides a molecular switch that is capable of starting the expression of the polynucleotide sequence operably linked to the inducible promoter upon expression, and terminating the expression when the expression is undesirable. Examples of inducible promoters include, but are not limited to, metallothionein promoters, glucocorticoid promoters, progesterone promoters, and tetracycline promoters.

In order to assess the expression of CAR polypeptides or portions thereof, the expression vectors introduced into the cells may also include one or both of the selectable marker genes or reporter genes to facilitate the identification and selection of expression cells from a cell population sought to be transfected or infected by a viral vector. In other aspects, the selectable markers may be carried on a single DNA segment and used for co-transfection procedures. The flanking regions of both selectable markers and reporter genes may have appropriate regulatory sequences to enable them to be expressed in host cells. Useful selectable markers include, for example, antibiotic resistance genes (such as nco).

Reporter genes are used for identifying potentially transfected cells and evaluating the functionality of regulatory sequences. After DNA has been introduced into recipient cells, the expression of the reporter gene is then measured at an appropriate time. Appropriate reporter genes may contain genes encoding luciferase, β-galactosidase, chloramphenicol acetyltransferase (CAT), secreted alkaline phosphatase (SAP), or green fluorescent protein gene (GFP). Appropriate expression systems are well-known and can be prepared using known techniques or commercially available techniques.

Methods of gene overexpression in cells are known in the art. The vector may be easily introduced into a host cell, e.g., a mammal, a bacterium, a yeast, or an insect cell, by any method in the art. For example, the expression vector may be transferred into a host cell by physical, chemical, or biological methods.

Physical methods of introducing polynucleotides into host cells include calcium phosphate precipitation, lipid transfection, particle bombardment, micro-injection, electroporation, etc. Biological methods of introducing polynucleotides of interest into host cells include the use of DNA and RNA vectors. Chemical methods of introducing polynucleotides into host cells include colloidal dispersion systems, such as macromolecular complexes, nanocapsules, microspheres, and beads; and lipid-based systems, including oil-in-water emulsions, micelles, mixed micelles, and liposomes.

Biological methods of introducing polynucleotides into host cells further include the use of viral vectors, in particular lentivirus vectors, which have become the most widely used methods of inserting genes into mammals, such as human cells. Other viral vectors may be derived from lentivirus, poxvirus, herpes simplex virus I, adenovirus, adeno-associated virus, etc. Many virus-based systems have been developed for transferring genes into mammal cells. For example, lentivirus provides a convenient platform for gene delivery systems. The selected genes can be inserted into the vector and packaged into lentivirus particles using techniques known in the art, and the obtained recombinant virus may then be separated and transferred to a subject cell in vivo or in vitro. Many retrovirus systems are known in the art. In some embodiments, an adenovirus vector is used. Many adenovirus vectors are known in the art. In an embodiment, a lentivirus vector is used.

The lentivirus vector system provided by the present disclosure includes the foregoing nucleic acid construct and a lentivirus vector auxiliary component.

The lentivirus auxiliary component includes a lentivirus packaging plasmid and a cell line.

The lentivirus vector system is formed by packaging the nucleic acid construct through a virus with the aid of the lentivirus packaging plasmid and the cell line. The method for constructing the lentivirus vector system is a common method in the art.

The present disclosure provides a method for activation of T cells in vitro, including: infecting the T cells with the aforementioned lentivirus.

Compared with other drug therapies, CAR-T cells can maintain in vivo anti-tumor function for a longer time due to their proliferation ability and viability. Once stimulated by the target-specific antigen, CAR-T cells amplify rapidly and can kill target tumor cells, then tumor-specific memory cells are formed, such as effector memory T cells and central memory T cells.

The present disclosure also provides a cell therapy, where T cells are genetically modified to express CAR described herein and CAR-T cells are injected into subjects in need thereof. The injected cells can kill tumor cells in subjects. Unlike antibody therapy, CAR-T cells can replicate in vivo, thus producing long-term control of the tumor.

The antitumor immune response caused by CAR-T cells may be an active or a passive immune response. In addition, the CAR-mediated immune response can be part of adoptive immunotherapy, where the CAR-T cell induces an immune response specifically corresponding to the antigen-binding moiety in CAR.

Cancer that can be treated may be a non-solid tumor, such as a blood tumor (such as leukemia and lymphoma). In particular, the diseases that can adopt the CAR and its coding sequence, nucleic acid construct, expression vector, virus, and CAR-T cell of the present disclosure for treatment are CD19-mediated diseases, especially CD19-mediated hematologic tumors.

Specifically, "CD19-mediated diseases" include, but are not limited to, leukemia and lymphoma, such as B-cell lymphoma, mantle cell lymphoma, acute lymphoblastic leukemia (ALL), chronic lymphocytic leukemia (CLL), hairy cell leukemia (HCL), and acute myeloid leukemia (AML).

The present disclosure provides the genetically modified T cell or pharmaceutical composition including the genetically modified T cell, where the genetically modified T cell includes the above polynucleotide sequence or nucleic acid construct, or infects the aforementioned lentivirus vector system.

The CAR-modified T cells of the present disclosure may be separately administrated or administrated as the pharmaceutical composition in combination with a diluent and/or other components (such as related cytokines or cell population). Briefly stated, the pharmaceutical composition of the present disclosure may include CAR-T cells described herein, which can be in combination with one or more medically or physiologically acceptable vectors, diluents, or excipients. Such composition may include buffer solutions, such as neutral buffer brine and sulfate buffer brine; carbohydrates, such as glucose, mannose, sucrose or glucan, and mannitol; protein; polypeptides or amino acids, such as glycine; antioxidant; chelating agent, such as EDTA or glutathione; adjuvant (such as aluminium hydroxide); and antiseptic agent.

The pharmaceutical composition of the present disclosure may be administrated in the way required by diseases to be treated (or prevented). The amount and frequency of administration depend on such factors, for example, the patient's symptoms, and disease type and severity.

When the "immunologically effective dosage", "antitumor effective dosage", "tumor-inhibitory effective dosage" or "therapeutic dosage" are indicated, the precise dosage of the composition of the present disclosure to be administrated depends on physicians after taking into account the age, weight, tumor size, infection or metastasis degree of the patient (subject) and individual differences. It may be indicated that the pharmaceutical composition containing the T cells described herein may be administrated at the dose of 10⁴ to 10⁹ cells/kg body weight, preferably 10⁵ to 10⁶ cells/kg body weight. The composition containing the T-cell may also be administrated multiple times at these doses. The cells may be administered by injection techniques known in the field of immunotherapy. The optimal dose and therapy scheme for a particular patient may be readily determined by a person skilled in the medical field by monitoring the signs of disease of the patient which facilitates the adjustment of the treatment.

**The** administration of the composition may be performed in any convenient manner, including spray, injection, swallowing, infusion, implantation, or transplantation. The compositions described herein may be administrated to patients subcutaneously, intracutaneously, intratumorally, nodally, intraspinally, intramuscularly, intravenously, or intraperitoneally. In an embodiment, the composition containing the T cell of the present disclosure is administrated to patients through intracutaneous or subcutaneous injection. In another embodiment, the composition containing the T cell of the present disclosure is administrated by intravenous injection. The composition containing the T cell may be injected directly into a tumor, lymph gland, or infection site.

In some embodiments of the present disclosure, the CAR-T cells or the composition thereof of the present disclosure may be combined with other therapies known in the art. The therapies include, but are not limited to, chemotherapy, radiotherapy, and immunosuppressants. For example, the CAR-T cells or the composition thereof of the present disclosure may be administrated in combination with various radiotherapy formulations, including cyclosporine, thiopurine, methotrexate, mycophenolate mofetil, FK506, fludarabine, rapamycin, mycophenolic acid, etc. In a further embodiment, the composition containing the T cell of the present disclosure is administered to the patient (e.g., before, during, or after) with bone marrow transplantation; a chemotherapeutic agent, such as fludarabine; external radiotherapy (XRT), cyclophosphamide or antibody, such as OKT3; or CAMPATH's T cell ablation therapy.

"Anti-tumor effect" herein refers to a biological effect that may be represented as a reduction in tumor size, a reduction in the number of tumor cells, a reduction in the number of metastasis, an increase in expected lifetime, or an alleviation of various physiological symptoms associated with cancer.

"Patient", "object", "individual", and the like may be used interchangeably herein to refer to a living organism, such as a mammal, that may cause an immune response. Examples include, but are not limited to, humans, dogs, cats, mice, rats, and transgenic species thereof.

**The** use of the aforementioned chimeric antigen receptor, polynucleotide sequence, nucleic acid construct, and lentivirus vector system in any one or more of the following: (1) preparing T cells; (2) enhancing T cells viability; (3) inhibiting T cells from apoptosis; (4) enhancing T cell proliferation capacity; (5) improving T cell anti-tumor capacity; (6) inhibiting T cells from secreting cytokines IFN-γ and TNF-α.

**An** increase in T cell viability, a reduction in T cell apoptosis, and an enhancement in T cell proliferation capacity contribute to the survival and persistence of T cells, thereby improving the anti-tumor capacity of T cells.

**The** use of the aforementioned chimeric antigen receptor, polynucleotide sequence, nucleic acid construct, and lentivirus vector system in any one or more of the following: enhancing the proliferation capability and viability of T cells, enhancing growth and proliferation ability or continuous proliferation ability of T cells, or increasing the number of T cells.

The use of the above chimeric antigen receptor, polynucleotide sequence, nucleic acid construct, lentivirus vector system, or genetically modified T cell in the preparation of tumor treatment products.

Optionally, the tumor is selected from leucocythemia or one or more of solid tumors.

Optionally, the tumor is selected from B-cell lymphoma, mantle cell lymphoma, acute lymphoblastic leukemia (ALL), chronic lymphocytic leukemia (CLL), hairy cell leukemia (HCL), and acute myeloid leukemia (AML).

The implementations of the present disclosure are described below through specific embodiments. Other advantages and effects of the present disclosure will be readily apparent to those skilled in the art from the content disclosed in the description. The present disclosure can also be implemented or applied through other different specific embodiments, and various details in the description can also be modified or changed without departing from the spirit of the present disclosure based on different viewpoints and applications.

Before further describing the specific embodiments of the present disclosure, it should be understood that the protection scope of the present disclosure is not limited to the specific embodiments described below. It should also be understood that the terms used in the embodiments of the present disclosure are intended to describe specific embodiments, rather than limiting the protection scope of the present disclosure. As used herein, "a", "an", and "the" in the singular form also include plural forms, unless otherwise stated herein.

When a numerical range is provided in an embodiment, it should be understood that any value between two endpoints of each numerical range and the two endpoints may be selected unless otherwise indicated by the present disclosure. Unless otherwise defined, all technical and scientific terms in the present disclosure have the same meaning as commonly understood by those skilled in the art. In addition to the specific methods, apparatuses, and materials used in the embodiments, any method, apparatus, and material of the prior art similar to or equivalent to those described in the embodiments of the present disclosure may also be used.

Unless otherwise specified, the experimental methods, detection methods, and preparation methods disclosed in the present disclosure are conventional techniques in molecular biology, biochemistry, chromatin structure and analysis, analytical chemistry, cell culture, recombinant DNA technology, and other related fields.

### Embodiment 1

Anti-CD19 28Z CAR sequence includes FMC63 scFv, CD28 hinge region, CD28 transmembrane region, CD28 and CD3ζ intracellular region structure, which is shared by Rosenberg, S.A. laboratory on the NCBI (GenBank: HM852952.1). Anti-CD19 28Z CAR was obtained by gene synthesis, and the CAR structure was designed as shown in Fig. 1a. The eGFP and CAR sequences were connected to the same open reading frame (ORF) by T2A self-cleaving peptide, so that the expression level of CAR can be indicated by the expression level/fluorescence intensity of eGFP. The CD3ε intracellular region with a Y/F mutation (both tyrosines in the ITAM of the intracellular region are mutated to phenylalanines) was inserted behind the CD28 transmembrane region by the site-directed mutagenesis based on overlap extension PCR and gene recombination technology, and a newly constructed CAR was obtained and named anti-CD19 E_{YF}28Z CAR.

The nucleotide sequence of the anti-CD19 E_{YF}28Z CAR is shown as SEQ ID NO: 8, specifically:

ATGGTGAGCAAGGGCGAGGAGCTGTTCACCGGGGTGGTGCCC
ATCCTGGTCGAGCTGGACGGCGACGTAAACGGCCACAAGTTCAGCGTG
TCCGGCGAGGGCGAGGGCGATGCCACCTACGGCAAGCTGACCCTGAA
GTTCATCTGCACCACCGGCAAGCTGCCCGTGCCCTGGCCCACCCTCGT
GACCACCCTGACCTACGGCGTGCAGTGCTTCAGCCGCTACCCCGACCA
CATGAAGCAGCACGACTTCTTCAAGTCCGCCATGCCCGAAGGCTACGTC
CAGGAGCGCACCATCTTCTTCAAGGACGACGGCAACTACAAGACCCGC
GCCGAGGTGAAGTTCGAGGGCGACACCCTGGTGAACCGCATCGAGCT
GAAGGGCATCGACTTCAAGGAGGACGGCAACATCCTGGGGCACAAGCT
GGAGTACAACTACAACAGCCACAACGTCTATATCATGGCCGACAAGCAG
AAGAACGGCATCAAGGTGAACTTCAAGATCCGCCACAACATCGAGGACG
GCAGCGTGCAGCTCGCCGACCACTACCAGCAGAACACCCCCATCGGCG
ACGGCCCCGTGCTGCTGCCCGACAACCACTACCTGAGCACCCAGTCCG
CCCTGAGCAAAGACCCCAACGAGAAGCGCGATCACATGGTCCTGCTGG
AGTTCGTGACCGCCGCCGGGATCACTCTCGGCATGGACGAGCTGTACA
AGGGAAGCGGAGAGGGCAGAGGAAGTCTGCTAACATGCGGTGACGTC
GAGGAGAATCCTGGACCTatgcttctcctggtgacaagccttctgctctgtgagttaccacaccc
agcattcctcctgatcccaTACCCCTACGACGTGCCCGACTACGCCgacatccagatga
cacagactacatcctccctgtctgcctctctgggagacagagtcaccatcagttgcagggcaagtcagga
cattagtaaatatttaaattggtatcagcagaaaccagatggaactgttaaactcctgatctaccatacatca
agattacactcaggagtcccatcaaggttcagtggcagtgggtctggaacagattattctctcaccattagc
aacctggagcaagaagatattgccacttacttttgccaacagggtaatacgcttccgtacacgttcggagg
ggggactaagttggaaataacaggctccacctctggatccggcaagcccggatctggcgagggatccac
caagggcgaggtgaaactgcaggagtcaggacctggcctggtggcgccctcacagagcctgtccgtca
catgcactgtctcaggggtctcattacccgactatggtgtaagctggattcgccagcctccacgaaagggtc
tggagtggctgggagtaatatggggtagtgaaaccacatactataattcagctctcaaatccagactgacc
atcatcaaggacaactccaagagccaagttttcttaaaaatgaacagtctgcaaactgatgacacagcca

tttactactgtgccaaacattattactacggtggtagctatgctatggactactggggtcaaggaacctcagtc
accgtctcctcagcggccgcaattgaagttatgtatcctccttacctagacaatgagaagagcaatgga
accattatccatgtgaaagggaaacacctttgtccaagtcccctatttcccggaccttctaagcccttttgggt
gctggtggtggttgggggagtcctggcttgctatagcttgctagtaacagtggcctttattattttctgggtgaag
aatagaaaggccaaggccaagcctgtgacacgaggagcgggtgctggcggcaggcaaaggggaca
aaacaaggagaggccaccacCTGTTCCCAACCCAGACTTTGAGCCCATCCGGA
AAGGCCAGCGGGACCTGTTTTCTGgcctgaatcagagacgcatcaggagtaagagga
gcaggctcctgcacagtgactacatgaacatgactccccgccgccccgggcccacccgcaagcattacc
agccctatgccccaccacgcgacttcgcagcctatcgctccagagtgaagttcagcaggagcgcagacg
cccccgcgtaccagcagggccagaaccagctctataacgagctcaatctaggacgaagagaggagta
cgatgttttggacaagagacgtggccgggaccctgagatggggggaaagccgagaaggaagaaccct
caggaaggcctgtacaatgaactgcagaaagataagatggcggaggcctacagtgagattgggatgaa
aggcgagcgccggaggggcaaggggcacgatggcctttaccagggtctcagtacagccaccaaggac
acctacgacgcccttcacatgcaggccctgccccctcgctaa.

**An** anti-CD19 E28Z CAR is obtained based on the method for constructing E_{YF}28Z. The only difference is that the CD3ε intracellular region with a Y/F mutation is replaced by CD3ε intracellular region. The nucleotide sequence of anti-CD19 E28Z CAR is shown as SEQ ID NO: 9, specifically:

ATGGTGAGCAAGGGCGAGGAGCTGTTCACCGGGGTGGTGCCC
ATCCTGGTCGAGCTGGACGGCGACGTAAACGGCCACAAGTTCAGCGTG
TCCGGCGAGGGCGAGGGCGATGCCACCTACGGCAAGCTGACCCTGAA
GTTCATCTGCACCACCGGCAAGCTGCCCGTGCCCTGGCCCACCCTCGT
GACCACCCTGACCTACGGCGTGCAGTGCTTCAGCCGCTACCCCGACCA
CATGAAGCAGCACGACTTCTTCAAGTCCGCCATGCCCGAAGGCTACGTC
CAGGAGCGCACCATCTTCTTCAAGGACGACGGCAACTACAAGACCCGC
GCCGAGGTGAAGTTCGAGGGCGACACCCTGGTGAACCGCATCGAGCT
GAAGGGCATCGACTTCAAGGAGGACGGCAACATCCTGGGGCACAAGCT
GGAGTACAACTACAACAGCCACAACGTCTATATCATGGCCGACAAGCAG
AAGAACGGCATCAAGGTGAACTTCAAGATCCGCCACAACATCGAGGACG
GCAGCGTGCAGCTCGCCGACCACTACCAGCAGAACACCCCCATCGGCG
ACGGCCCCGTGCTGCTGCCCGACAACCACTACCTGAGCACCCAGTCCG
CCCTGAGCAAAGACCCCAACGAGAAGCGCGATCACATGGTCCTGCTGG

AGTTCGTGACCGCCGCCGGGATCACTCTCGGCATGGACGAGCTGTACA
AGGGAAGCGGAGAGGGCAGAGGAAGTCTGCTAACATGCGGTGACGTC
GAGGAGAATCCTGGACCTatgcttctcctggtgacaagccttctgctctgtgagttaccacaccc
agcattcctcctgatcccaTACCCCTACGACGTGCCCGACTACGCCgacatccagatga
cacagactacatcctccctgtctgcctctctgggagacagagtcaccatcagttgcagggcaagtcagga
cattagtaaatatttaaattggtatcagcagaaaccagatggaactgttaaactcctgatctaccatacatca
agattacactcaggagtcccatcaaggttcagtggcagtgggtctggaacagattattctctcaccattagc
aacctggagcaagaagatattgccacttacttttgccaacagggtaatacgcttccgtacacgttcggagg
ggggactaagttggaaataacaggctccacctctggatccggcaagcccggatctggcgagggatccac
caagggcgaggtgaaactgcaggagtcaggacctggcctggtggcgccctcacagagcctgtccgtca
catgcactgtctcaggggtctcattacccgactatggtgtaagctggattcgccagcctccacgaaagggtc
tggagtggctgggagtaatatggggtagtgaaaccacatactataattcagctctcaaatccagactgacc
atcatcaaggacaactccaagagccaagttttcttaaaaatgaacagtctgcaaactgatgacacagcca
tttactactgtgccaaacattattactacggtggtagctatgctatggactactggggtcaaggaacctcagtc
accgtctcctcagcggccgcaattgaagttatgtatcctcctccttacctagacaatgagaagagcaatgga
accattatccatgtgaaagggaaacacctttgtccaagtcccctatttcccggaccttctaagcccttttgggt
gctggtggtggttgggggagtcctggcttgctatagcttgctagtaacagtggcctttattattttctgggtgaag
aatagaaaggccaaggccaagcctgtgacacgaggagcgggtgctggcggcaggcaaaggggaca
aaacaaggagaggccaccacctgttcccaacccagactatgagcccatccggaaaggccagcggga
cctgtattctggcctgaatcagagacgcatcaggagtaagaggagcaggctcctgcacagtgactacatg
aacatgactccccgccgccccgggcccacccgcaagcattaccagccctatgccccaccacgcgacttc
gcagcctatcgctccagagtgaagttcagcaggagcgcagacgcccccgcgtaccagcagggccaga
accagctctataacgagctcaatctaggacgaagagaggagtacgatgttttggacaagagacgtggcc
gggaccctgagatggggggaaagccgagaaggaagaaccctcaggaaggcctgtacaatgaactgc
agaaagataagatggcggaggcctacagtgagattgggatgaaaggcgagcgccggaggggcaag
gggcacgatggcctttaccagggtctcagtacagccaccaaggacacctacgacgcccttcacatgcag
gccctgccccctcgctaa.

**The** CAR was subcloned to a lentivirus expression plasmid pHAGE vector, and the CAR expression plasmid, the packaging plasmid pSPAX2, and the pMD2G were mixed at a ratio of 10: 7.5: 3.5, afterward, were transferred into 293 FT cells by a calcium phosphate transfection method to produce lentivirus particles. The lentiviruses expressing anti-CD19 28Z CAR, anti-CD19 E28Z CAR, or anti-CD19 E_{YF}28Z CAR were concentrated into small-volume high-titer viruses (^{~}10⁸ IU/ml) by ultracentrifugation method, and then were used to transfect primary T cells (MOI=10) activated by αCD3/αCD28 Ab-beads for one day. The primary T cells were cultured with T cell complete medium (XIVO-15+1% P.S.+10ng/ml human IL-7+10ng/ml human IL-15+5% human AB serum, 10 mM neutralized NAC), the viruses were removed one day after transfection, and the antibodies were removed four days after stimulation. On the 5th day after antibody stimulation, CAR-T cells with the same CAR expression level (represented by eGFP fluorescence intensity) were sorted out. The obtained T-cells were anti-CD19 28Z CAR-T cells, anti-CD19 E28Z CAR-T cells, and anti-CD19 E_{YF}28Z CAR-T cells. After a period of amplification, CAR-T was characterized by a series of indicators, such as surface CAR level, CD4/CD8 ratio, in vitro cytokine secretion, in vitro killing ability, in vitro proliferation ability, in vitro apoptosis level, in vitro sustained viability, and in vivo anti-tumor effect.

The indicator detection methods and results are shown as follows.

The structures of the above anti-CD19 28Z CAR, anti-CD19 E28Z CAR, and anti-CD19 E_{YF}28Z CAR, the amino acid sequence of the CD3ε intracellular region, and the amino acid sequence of the CD3ε intracellular region with a Y/F mutation are shown in Fig. 1a. FMC63 is a single-chain antibody targeting CD19 antigen, and the hinge region and transmembrane region of the receptor derive from human CD28. In anti-CD19 E28Z CAR, CD3ε is inserted behind the CD28 transmembrane region and before the CD28 intracellular region. In anti-CD19 E_{YF}28Z CAR, the amino acid sequence of the CD3ε intracellular region with a Y/F mutation is inserted behind the CD28 transmembrane region and before the CD28 intracellular region.

**The** CAR levels on the membrane surface of the anti-CD19 28Z CAR-T cells, anti-CD19 E28Z CAR-T cells, and anti-CD19 E_{YF}28Z CAR-T cells were detected by flow cytometry employing anti-mouse FMC63 scFv coupled with Alexa Fluor 647 antibody. The results of the flow cytometry are shown in Fig. 1b, indicating that there was no difference in CAR positive rate and expression level (MFI) among the anti-CD19 28Z CAR-T cells, anti-CD19 E28Z CAR-T cells, and anti-CD19 E_{YF}28Z CAR-T cells.

The anti-CD19 28Z CAR-T cells, anti-CD19 E28Z CAR-T cells, and anti-CD19 E_{YF}28Z CAR-T cells were detected by flow cytometry employing anti-human CD4-APC and anti-human CD8-PE-Cy7 antibodies. The results of the flow cytometry are shown in Fig. 1c, indicating that the ratios of CD4⁺ CAR-T cells to CD8⁺ CAR-T cells of the anti-CD19 28Z CAR-T cells, anti-CD19 E28Z CAR-T cells, and anti-CD19 E_{YF}28Z CAR-T cells were similar.

100,000 anti-CD19 28Z CAR-T cells, anti-CD19 E28Z CAR-T cells, and anti-CD19 E_{YF}28Z CAR-T cells were respectively inoculated with CD19⁺ lymphoma cell line Raji in round-bottom 96-well plates at ratios of 1:1, then mixed well, and afterward, centrifuged (400 g, 1 min) at room temperature to promote cell-cell contact. The obtained samples were cultured with complete mediums putting in a 37°C incubator for one day before sample collection. 1x BFA was added to each sample 6 hours before sample collection to inhibit the efflux of cytokine, and then the collected samples were washed once with PBS, afterward, were immobilized at room temperature for 5 min using 4% PFA, then were punched at room temperature for 5 min utilizing 0.1% TritonX-100. Next, IL-2, IFN-γ, and TNF-α were detected using the direct labeling of antibodies according to intracellular staining known in the art. As shown in Fig. 1d - Fig. 1f, compared with anti-CD19 E28Z CAR-T cells, the cytokines IL-2, IFN-γ, and TNF-α produced by anti-CD19 E_{YF}28Z CAR-T cells after being specifically stimulated by CD19⁺ cells was significantly increased, indicating that the ITAM of the CD3ε intracellular region has an important inhibitory effect on the production of cytokines. However, it is noteworthy that the cytokines IFN-γ and TNF-α secreted by anti-CD19 E_{YF}28Z CAR-T cells were still significantly less than those of anti-CD19 28Z CAR-T cells (Fig. 1e - Fig. 1f), manifesting the anti-CD19 E_{YF}28Z CAR-T cells can reduce the activation of macrophages and monocytes to produce inflammatory cytokines such as IL-1 β and IL-6.

100,000 anti-CD19 28Z CAR-T cells, anti-CD19 E28Z CAR-T cells, and anti-CD19 E_{YF}28Z CAR-T cells were respectively inoculated with CD19⁺ lymphoma cell line Raji in round-bottom 96-well plates at ratios of 1:1, then mixed well, afterward, centrifuged (400 g, 1 min) at room temperature to promote cell-cell contact. The obtained samples were cultured with complete mediums putting in a 37°C incubator for a period of time. After sample collection at different time points, the collected samples were washed once with PBS, respectively. According to the instructions of the Foxp3/Transcription Factor Staining Buffer Set Kit, the Ki67 expression levels were detected by flow cytometry after anti-human Ki67-APC antibody staining. As shown in Fig. 1g, the proliferation rate of anti-CD19 E_{YF}28Z CAR-T after being stimulated by CD19⁺ Raji cells was faster than that of anti-CD19 28Z CAR-T cells, and even faster than that of anti-CD19 E28Z CAR-T cells during a period of time, that is, anti-CD19 E_{YF}28Z CAR-T cells had the strongest proliferation ability.

100,000 anti-CD19 28Z CAR-T cells, anti-CD19 E28Z CAR-T cells, and anti-CD19 E_{YF}28Z CAR-T cells were respectively inoculated with CD19⁺ lymphoma cell line Raji in round-bottom 96-well plates at ratios of 1:1, then mixed well, and afterward, centrifuged (400 g, 1 min) at room temperature to promote cell-cell contact. The obtained samples were cultured with complete mediums putting in a 37°C incubator for a period of time. After sample collection at different time points, the collected samples were washed once with PBS, respectively. According to the instructions of the Annexin V Apoptosis Detection Kit APC Kit, the Annexin V expression levels of CAR-T cells were detected by flow cytometry after staining. As shown in Fig. 1h, the apoptosis levels of anti-CD19 E_{YF}28Z CAR-T cells and anti-CD19 E28Z CAR-T cells after being stimulated by CD19⁺ Raji cells were significantly lower than that of anti-CD19 28Z CAR-T cells during a period of time.

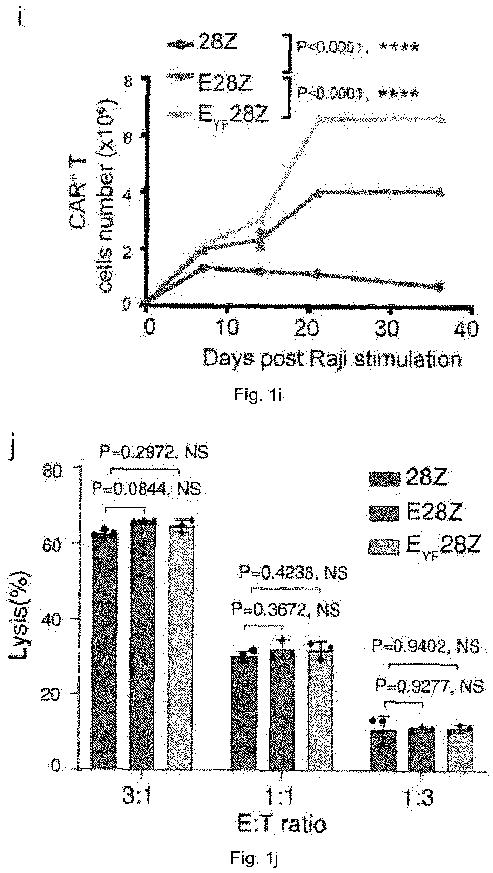

100,000 anti-CD19 28Z CAR-T cells, anti-CD19 E28Z CAR-T cells, and anti-CD19 E_{YF}28Z CAR-T cells were respectively inoculated with CD19⁺ lymphoma cell line Raji in round-bottom 96-well plates at ratios of 1:1, then mixed well, afterward, were centrifuged (400 g, 1 min) at room temperature to promote the cell-cell contact. The obtained samples were cultured with complete mediums putting in a 37°C incubator for a period of time. After collecting the samples at different time points, the collected samples were washed with PBS once respectively, and the survival amount of CAR⁺T cells was detected with anti-human CD4-APC and anti-human CD8-PE-Cy7 antibody staining. The results are shown in Fig. 1i, after stimulation of CD19⁺ Raji cells, the survival amount of anti-CD19 E_{YF}28Z CAR-T cells was the highest, followed by anti-CD19 E28Z CAR-T cells, and the survival amount of anti-CD19 28Z CAR-T cells was the lowest, which indicates that the sustained viability of E_{YF}28Z CAR-T cells was the highest.

Cytotoxicity of anti-CD19 28Z CAR-T cells, anti-CD19 E28Z CAR-T cells, and anti-CD19 E_{YF}28Z CAR-T cells against CD19⁺ tumor cells. CD19⁻K562 cells were resuspended in a 1640 serum-free medium, and then pre-stained with 1x CellTrader deep red dye (37°C water bath, 30min), next were washed with 1640 serum-free medium once, and afterward were resuspended in complete T cell medium. The resuspended CD19⁻K562 cells were mixed with CD19⁺K562_CD19 (IRES mCherry) cells at a ratio of 1:1 to serve as target cells. Anti-CD19 CAR-T cells were mixed with the above target cells in around-bottom 96-well plates at ratios of 3:1, 1:1, and 1:3, respectively, then mixed well, and afterward centrifuged (400g, 1min) at room temperature to promote cell-cell contact. The obtained samples were cultured with complete mediums putting in a 37°C incubator for one day. The samples were collected and placed in ice. The percentage of CD19⁻K562 cells (APC⁺, mCherrγ) and the percentage of CD19⁺K562_ CD19 cells (APC⁻, mCherry⁺) were detected by flow cytometry, where "the percentage of CD19⁻K562 cells (APC⁺, mCherrγ) / the percentage of CD19⁺K562_CD19 cells (APC⁻, mCherry⁺)" in the experimental group was recorded as N. N was standardized with N₀, where "N₀= the percentage of CD19K562 cells (APC⁺, mCherrγ) / the percentage of CD19⁺K562_CD19 cells (APC⁻, mCherry⁺)" of the target cells in which no CAR-T cells were added. The survival ratio was calculated as N/N₀ after standardization, and the killing rate formula was "Lysis (%)=1-N/N₀". As shown in Fig. 1j, anti-CD19 28Z CAR-T cells, anti-CD19 E28Z CAR-T cells, and anti-CD19E_{YF}28Z CAR-T cells had similar cytotoxicity against CD19⁺ tumor cells.

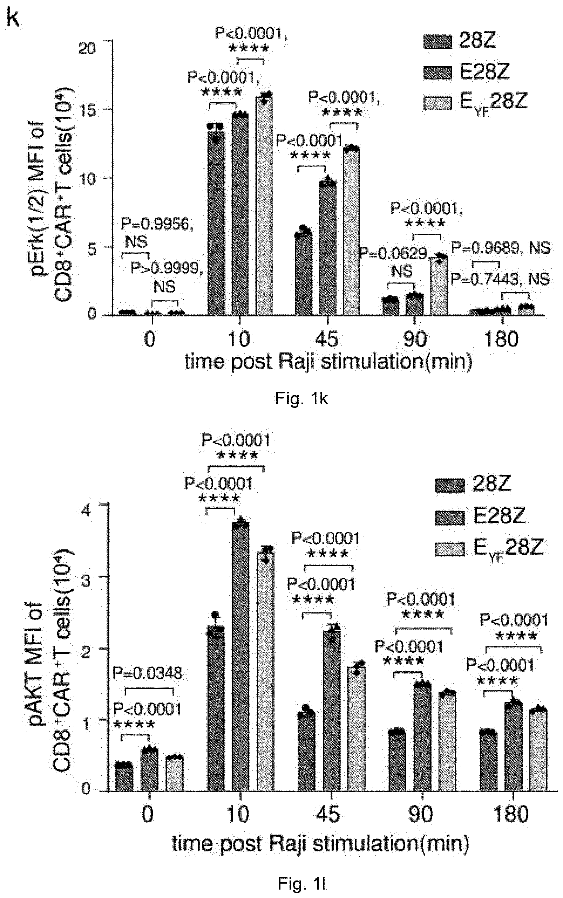

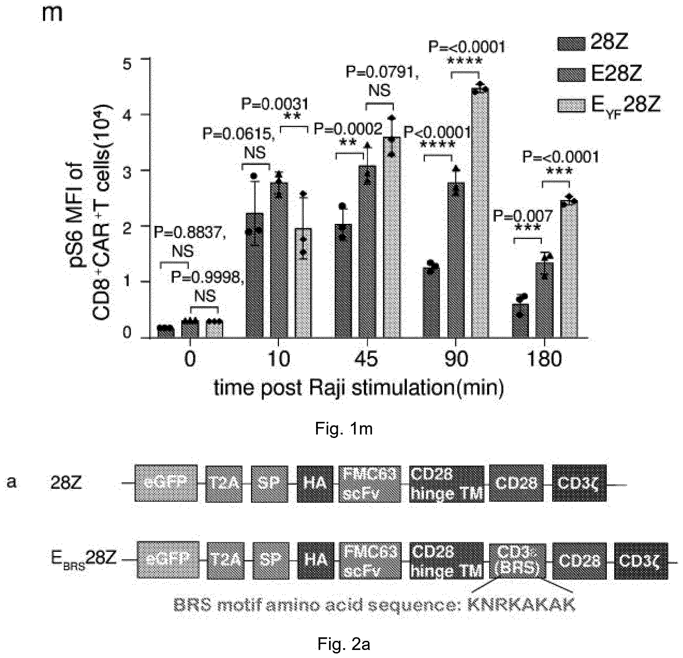

100,000 anti-CD19 28Z CAR-T cells, anti-CD19 E28Z CAR-T cells, and anti-CD19 E_{YF}28Z CAR-T cells were inoculated with the CD19⁺ lymphoma cell line Raji at ratios of 1:1 in 1.5ml EP tubes, mixed well, centrifuged (500g, 2min) at 4°C to promote cell-cell contact, and cultured with complete mediums put in a 37°C water bath. 8% PFA with the same volumes were added at different time points for immobilization at room temperature for 5min, and then the samples were punched using 0.1% TritonX-100 at room temperature for 5min. Then, according to the intracellular staining known in the art, pErk(1/2)^{Thr202/Tyr204} rabbit antibody (Fig. 1k), pAKT^{S473} rabbit antibody (Fig. 1l), and pS6^{S235/236} rabbit antibody (Fig. 1m) were first used as the primary antibodies to stain and washed once, latter goat anti-rabbit IgG labeled with Alexa647 were used as the second antibodies to stain and washed once, and afterward the flow cytometry was performed. As shown in Fig. 1k - Fig. 1m, the overall levels of pErk (1/2)^{Thr202/Tyr204} and pS6^{S235/236} of anti-CD19 E_{YF}28Z CAR-T cells after being specifically stimulated by antigen were significantly higher than those of anti-CD19 28Z CAR-T cells and anti-CD19 E28Z CAR-T cells, and the AKT^{S473} phosphorylation levels of anti-CD19 E_{YF}28Z CAR-T cells and anti-CD19 E28Z CAR-T cells were significantly higher than that of anti-CD19 28Z CAR-T cells. In the signal transduction pathway of the chimeric antigen receptor CAR activated by antigen, the phosphorylation level of the signaling molecule serine/threonine protein kinase Erk(1/2)^{Thr202/Tyr204} in the Raf/MAPK signaling pathway and the phosphorylation level of serine/threonine kinase AKT^{S473} in the PI3K/AKT signaling pathway indicate the ability to promote cell proliferation, viability, and anti-apoptosis. The phosphorylation level of the downstream ribosomal protein S6^{s235/236} of the above two pathways represents the level of cell growth and proliferation. The phenotypes of these three signaling molecules illustrate the strongest proliferation and viability of anti-CD19 E_{YF}28Z CAR-T cells from the perspective of signaling pathway.

Experimental data processing methods are shown as follows.

**The** software GraphPad Prism 8.0.2 was used for statistical data analysis. When P<0.05, the difference was statistically significant, specifically expressed as * P<0.05, ** P<0.01, *** P<0.001, **** P<0.0001, and each group of data was expressed in Mean ± SD.

Fig. 1d - Fig. 1f: one-way ANOVA with Sidak's multiple comparison.

Fig. 1g, Fig. 1h, Fig. 1j - Fig. 1m: two-way ANOVA with Sidak's multiple comparison.

Fig. 1i: two-way ANOVA.

Experimental results are shown as follows.
**(1)** The surface anti-CD19 CAR levels and the CD4/CD8 ratios of anti-CD19 E_{YF}28Z CAR-T cells, anti-CD19 E28Z CAR-T cells, and anti-CD19 28Z CAR-T cells were similar.
(2) After anti-CD19 28Z CAR-T cells, anti-CD19 E28Z CAR-T cells, and anti-CD19 EYF28Z CAR-T cells were specifically stimulated by Raji cells in vitro, the increase extent of cytokines IL-2, IFN-γ, and TNF-α produced by anti-CD19 E_{YF}28Z CAR-T cells were higher than that of anti-CD19 28Z CAR-T cells, indicating that ITAM of the CD3ε intracellular region has an important inhibitory effect on the production of cytokines. However, it is noteworthy that the above cytokines secreted by anti-CD19 E_{YF}28Z CAR-T cells were still less than those secreted by anti-CD19 28Z CAR-T cells (Fig. 1e - Fig. 1f), therefore, the anti-CD19 E_{YF}28Z CAR-T cells can reduce the activation of macrophages and monocytes to produce inflammatory cytokines such as IL-1β And IL-6.
(3) After anti-CD19 28Z CAR-T cells, anti-CD19 E28Z CAR-T cells, and anti-CD19 E_{YF}28Z CAR-T cells were specifically stimulated by Raji cells in vitro and amplified for a period of time, the proliferation abilities (Ki67 expression level) were detected at different time points, and the proliferation ability of anti-CD19 E_{YF}28Z CAR-T cells was higher than those of anti-CD19 28Z CAR-T cells and anti-CD19 E28Z CAR-T cells.
(4) After anti-CD19 28Z CAR-T cells, anti-CD19 E28Z CAR-T cells, and anti-CD19 E_{YF}28Z CAR-T cells were specifically stimulated by Raji cells in vitro and amplified for a period of time, the apoptosis levels (represented by Annexin V) were detected at different time points, and the apoptosis levels of anti-CD19 E28Z CAR-T cells and anti-CD19 E_{YF}28Z CAR-T cells were significantly lower than that of anti-CD19 28Z CAR-T cells.
(5) After anti-CD19 28Z CAR-T cells, anti-CD19 E28Z CAR-T cells, and anti-CD19 E_{YF}28Z CAR-T cells were specifically stimulated by Raji cells in vitro and amplified for a period of time, the number of amplified CAR+ T cells was detected at different time points, the number of anti-CD19 E_{YF}28Z CAR-T cells was consistently higher than those of anti-CD19 28Z CAR-T cells and anti-CD19 E28Z CAR-T cells.
(6) In the in vitro killing experiment where anti-CD19 28Z CAR-T cells, anti-CD19 E28Z CAR-T cells, and anti-CD19 EBRS28Z CAR-T cells were co-incubated with CD19⁺K562:CD19⁻K562, the killing ability of anti-CD19 E_{YF}28Z CAR-T cells was similar to those of anti-CD19 28Z CAR-T cells and anti-CD19 E28ZCAR-T cells.
(7) After anti-CD19 28Z CAR-T cells, anti-CD19 E28Z CAR-T cells, and anti-CD19 E_{YF}28Z CAR-T cells were specifically stimulated by Raji cells in vitro and amplified for a period of time, the phosphorylation levels of the signaling molecule serine/threonine protein kinase Erk(1/2)^{Thr202/Tyr204}, serine/threonine kinase AKT^{S473}, and ribosomal protein S6^{S235/236} were detected at different time points. The overall levels of pErk(1/2)^{Thr202/Tyr204} and pS6^{S235/236} of anti-CD19 E_{YF}28Z CAR-T cells after being specifically stimulated by antigen were significantly higher than those of anti-CD19 28Z CAR-T cells and anti-CD19 E28Z CAR-T cells, and the AKT^{S473} phosphorylation levels of anti-CD19 E_{YF}28Z CAR-T cells and anti-CD19 E28Z CAR-T cells were significantly higher than that of anti-CD19 28Z CAR-T cells. In the signal transduction pathway of chimeric antigen receptor CAR activated by antigen, the phosphorylation level of Erk(1/2)^{Thr202/Tyr204} signaling molecule in Raf/MAPK signaling pathway and the phosphorylation level of AKT^{S473} in PI3K/AKT signaling pathway indicate the ability to promote cell proliferation, viability, and anti-apoptosis, and the phosphorylation level of the downstream signaling molecule ribosomal protein S6^{S235/236} of the above two pathways manifests the level of cell growth and proliferation. The phenotypes of these three signaling molecules illustrate that anti-CD19 E_{YF}28Z CAR-T cells had the strongest proliferation and viability from the perspective of signaling pathway.

For the comparison of in vivo anti-subcutaneous tumor ability among anti-CD19 E_{YF}28Z CAR-T cells, anti-CD19 E28Z cells, and anti-CD19 28Z CAR-T cells, the experimental scheme is as follows:

3×10^7/ml Raji cell-PBS resuspension was prepared in a sterile condition, 100 ul (3 million) Raji cells were inoculated subcutaneously to the left back of each 6-week-old B-NDG female mouse, and the time of inoculation was denoted as day0. Six days later, the diameter of the Raji subcutaneous tumor became 7-8 mm. The mice then were randomly divided into four groups (E_{YF}28Z group, E28Z group, 28Z group, vector group), afterward, 100ul (8 million) T cells were injected into the tail vein of each mouse. After that, the size of the tumor was measured regularly with a vernier caliper, and the tumor data and survival period of the mice were recorded. The tumor area = the length x the width. The experimental conclusion is anti-CD19 E_{YF}28Z CAR-T cells have an anti-tumor effect.

### Embodiment 2

Anti-CD19 28Z CAR sequence includes FMC63 scFv, CD28 hinge region, CD28 transmembrane region, and CD3ζ intracellular region, which is shared by Rosenberg, S.A. laboratory on the NCBI (GenBank: HM852952.1). Anti-CD19 28Z CAR was obtained by gene synthesis, and the CAR structure was designed as shown in Fig. 2a. The eGFP and CAR sequences were connected to the same open reading frame (ORF) by T2A self-cleaving peptide, so that the expression level of CAR can be indicated by the expression level/fluorescence intensity of eGFP. BRS motif of the CD3ε intracellular segment was inserted behind the CD28 transmembrane region by using the overlap extension PCR technology, and a newly constructed CAR was obtained and named anti-CD19 E_{BRS}28Z CAR.

The nucleotide sequence of the anti-CD19 E_{BRS}28Z CAR is shown as SEQ ID NO: 13, specifically:

ATGGTGAGCAAGGGCGAGGAGCTGTTCACCGGGGTGGTGCCC
ATCCTGGTCGAGCTGGACGGCGACGTAAACGGCCACAAGTTCAGCGTG

TCCGGCGAGGGCGAGGGCGATGCCACCTACGGCAAGCTGACCCTGAA
GTTCATCTGCACCACCGGCAAGCTGCCCGTGCCCTGGCCCACCCTCGT
GACCACCCTGACCTACGGCGTGCAGTGCTTCAGCCGCTACCCCGACCA
CATGAAGCAGCACGACTTCTTCAAGTCCGCCATGCCCGAAGGCTACGTC
CAGGAGCGCACCATCTTCTTCAAGGACGACGGCAACTACAAGACCCGC
GCCGAGGTGAAGTTCGAGGGCGACACCCTGGTGAACCGCATCGAGCT
GAAGGGCATCGACTTCAAGGAGGACGGCAACATCCTGGGGCACAAGCT
GGAGTACAACTACAACAGCCACAACGTCTATATCATGGCCGACAAGCAG
AAGAACGGCATCAAGGTGAACTTCAAGATCCGCCACAACATCGAGGACG
GCAGCGTGCAGCTCGCCGACCACTACCAGCAGAACACCCCCATCGGCG
ACGGCCCCGTGCTGCTGCCCGACAACCACTACCTGAGCACCCAGTCCG
CCCTGAGCAAAGACCCCAACGAGAAGCGCGATCACATGGTCCTGCTGG
AGTTCGTGACCGCCGCCGGGATCACTCTCGGCATGGACGAGCTGTACA
AGGGAAGCGGAGAGGGCAGAGGAAGTCTGCTAACATGCGGTGACGTC
GAGGAGAATCCTGGACCTatgcttctcctggtgacaagccttctgctctgtgagttaccacaccc
agcattcctcctgatcccaTACCCCTACGACGTGCCCGACTACGCCgacatccagatga
cacagactacatcctccctgtctgcctctctgggagacagagtcaccatcagttgcagggcaagtcagga
cattagtaaatatttaaattggtatcagcagaaaccagatggaactgttaaactcctgatctaccatacatca
agattacactcaggagtcccatcaaggttcagtggcagtgggtctggaacagattattctctcaccattagc
aacctggagcaagaagatattgccacttacttttgccaacagggtaatacgcttccgtacacgttcggagg
ggggactaagttggaaataacaggctccacctctggatccggcaagcccggatctggcgagggatccac
caagggcgaggtgaaactgcaggagtcaggacctggcctggtggcgccctcacagagcctgtccgtca
catgcactgtctcaggggtctcattacccgactatggtgtaagctggattcgccagcctccacgaaagggtc
tggagtggctgggagtaatatggggtagtgaaaccacatactataattcagctctcaaatccagactgacc
atcatcaaggacaactccaagagccaagttttcttaaaaatgaacagtctgcaaactgatgacacagcca
tttactactgtgccaaacattattactacggtggtagctatgctatggactactggggtcaaggaacctcagtc
accgtctcctcagcggccgcaattgaagttatgtatcctcctccttacctagacaatgagaagagcaatgga
accattatccatgtgaaagggaaacacctttgtccaagtcccctatttcccggaccttctaagcccttttgggt
gctggtggtggttgggggagtcctggcttgctatagcttgctagtaacagtggcctttattattttctgggtgaag
aatagaaaggccaaggccaagaggagtaagaggagcaggctcctgcacagtgactacatgaacatg
actccccgccgccccgggcccacccgcaagcattaccagccctatgccccaccacgcgacttcgcagc
ctatcgctccagagtgaagttcagcaggagcgcagacgcccccgcgtaccagcagggccagaaccag

ctctataacgagctcaatctaggacgaagagaggagtacgatgttttggacaagagacgtggccgggac
cctgagatggggggaaagccgagaaggaagaaccctcaggaaggcctgtacaatgaactgcagaaa
gataagatggcggaggcctacagtgagattgggatgaaaggcgagcgccggaggggcaaggggcac
gatggcctttaccagggtctcagtacagccaccaaggacacctacgacgcccttcacatgcaggccctgc
cccctcgctaa

The CAR was subcloned into the lentivirus expression plasmid pHAGE vector, and the CAR expression plasmid was mixed with the packaging plasmid pSPAX2 and pMD2G in the ratio of 10:7.5:3.5, and then transferred into 293FT cells by a calcium phosphate transfection method to produce lentivirus particles. The lentiviruses expressing anti-CD19 28Z CAR and anti-CD19 E_{BRS}28Z CAR were concentrated into small volume high-titer viruses (~10⁸ IU/ml) by ultracentrifugation method, and then were used to transfect primary T cells (MOI=10) activated by αCD3/α CD28 Ab-beads for one day. The primary T cells were cultured with T cell complete medium (XIVO-15+1% P.S.+10ng/ml human IL-7+10ng/ml human IL-15+5% human AB serum, 10 mM neutralized NAC), the viruses were removed after one day after transfection, and the antibodies were removed four days after stimulation. The CAR-T cells with the same CAR expression level (represented by eGFP fluorescence intensity) were selected on the fifth day after antibody stimulation. The obtained T-cells were anti-CD19 28Z CAR-T cells and anti-CD19 E_{BRS}28Z CAR-T cells. After a period of amplification, CAR-T was characterized by a series of indicators, such as the surface CAR level, the CD4/CD8 ratio, the in vitro cytokine secretion, the in vitro killing ability, the in vitro proliferation ability, the in vitro apoptosis level, the in vitro sustained viability, and the in vivo anti-tumor effect.

The indicator detection methods and results are shown as follows.

The structures of the obtained anti-CD19 28Z CAR and anti-CD19 E_{BRS}28Z CAR are shown in Fig. 2a. FMC63 is a single-chain antibody targeting CD19 antigen, and the hinge region and transmembrane region of the receptor derive from human CD28. A human CD3ε intracellular basic residue rich sequence is inserted behind the CD28 transmembrane region and before the CD28 intracellular region.

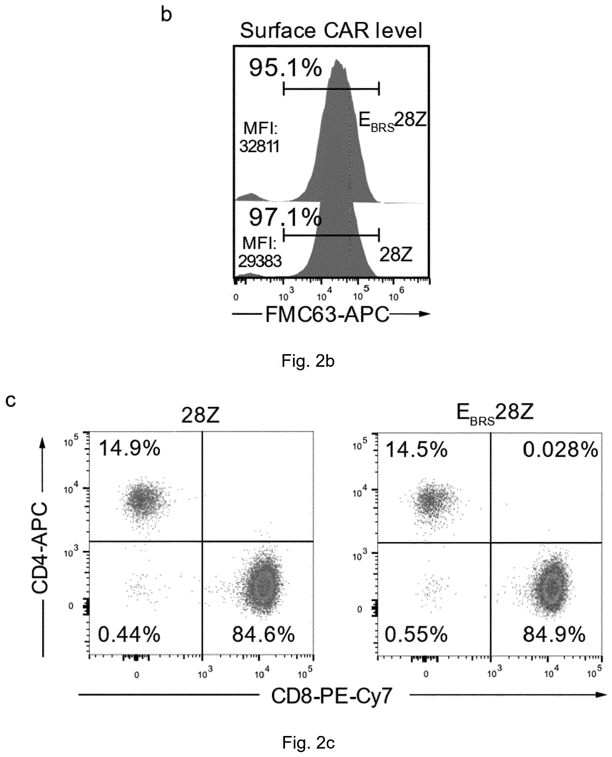

**The** CAR levels on the membrane surface of the anti-CD19 28Z CAR-T cells and anti-CD19 E_{BRS}28Z CAR-T cells were detected by flow cytometry employing anti-mouse FMC63 scFv coupled with Alexa Fluor 647 antibody. The results of the flow cytometry are shown in Fig. 2b, indicating there was no difference in CAR positive rate and expression level (MFI) between the anti-CD19 28Z CAR-T cells and anti-CD19 E_{BRS}28Z CAR-T cells.

The anti-CD19 28Z CAR-T cells and anti-CD19 E_{BRS}28Z CAR-T cells were detected by flow cytometry employing anti-human CD4-APC and anti-human CD8-PE-Cy7 antibodies. The results of the flow cytometry are shown in Fig. 2c, indicating that the CD4⁺/CD8⁺ ratios of the anti-CD19 28Z CAR-T cells and anti-CD19 E_{BRS}28Z CAR-T cells were similar.

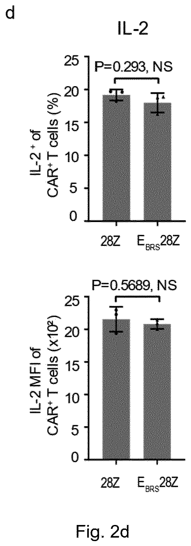

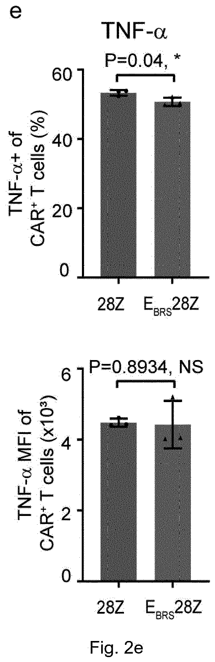

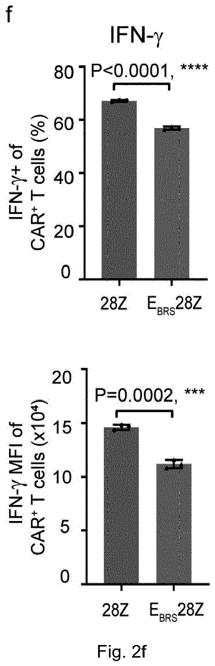

100,000 anti-CD19 28Z CAR-T cells and anti-CD19 E_{BRS}28Z CAR-T cells were respectively inoculated with CD19⁺ lymphoma cell line Raji in round-bottom 96-well plates at ratios of 1:1, then mixed well, afterward, were centrifuged (400 g, 1 min) at room temperature to promote cell-cell contact. The obtained samples were cultured with complete mediums putting in a 37°C incubator for one day before sample collection. 1x BFA was added to each sample 6 hours before sample collection to inhibit the efflux of cytokine, and then the collected samples were washed once with PBS, afterward, were immobilized at room temperature for 5 min using 4% PFA, then were punched at room temperature for 5 min utilizing 0.1% TritonX-100. Next, IL-2, IFN-γ, and TNF-α were detected using the direct labeling of antibodies according to intracellular staining known in the art. As shown in Fig. 2d, Fig. 2e, and Fig. 2f, the amount of cytokines IL-2 and TNF-α produced from anti-CD19 E_{BRS}28Z CAR-T cells after specific stimulation of CD19⁺ cells was similar to that of anti-CD19 28Z CAR-T cells, while the amount of cytokines IFN-γ was less than that of anti-CD19 28Z CAR-T cells.

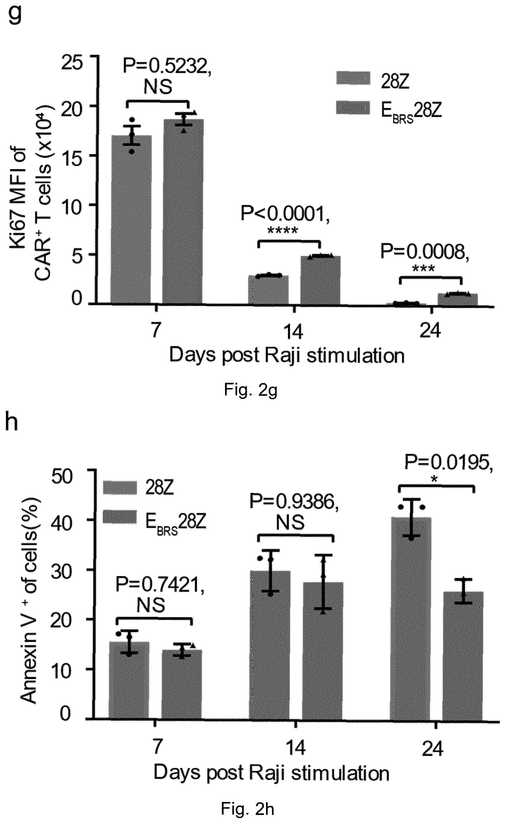

100,000 anti-CD19 28Z CAR-T cells and anti-CD19 E_{BRS}28Z CAR-T cells were respectively inoculated with CD19⁺ lymphoma cell line Raji in round-bottom 96-well plates at ratios of 1:1, then mixed well, afterward, were centrifuged (400 g, 1 min) at room temperature to promote cell-cell contact. The obtained samples were cultured with complete mediums putting in a 37°C incubator for a period of time. After sample collection at different time points, the collected samples were washed once with PBS, respectively. According to the instructions of the Foxp3/Transcription Factor Staining Buffer Set Kit, the Ki67 expression levels were detected by flow cytometry after anti-human Ki67-APC antibody staining. As shown in Fig. 2g, anti-CD19 E_{BRS}28Z CAR-T cells proliferated faster than anti-CD19 28Z CAR-T cells during a period of time after being stimulated by CD19⁺ Raji cells.

100,000 anti-CD19 28Z CAR-T cells and anti-CD19 E_{BRS}28Z CAR-T cells were respectively inoculated with CD19⁺ lymphoma cell line Raji in round-bottom 96-well plates at ratios of 1:1, then mixed well, afterward, were centrifuged (400 g, 1 min) at room temperature to promote cell-cell contact. The obtained samples were cultured with complete mediums putting in a 37°C incubator for a period of time. After sample collection at different time points, the collected samples were washed once with PBS, respectively. According to the instructions of the Annexin V Apoptosis Detection Kit APC Kit, the Annexin V expression levels of CAR-T cells were detected by flow cytometry after staining. As shown in Fig. 2h, the apoptosis level of anti-CD19 E_{BRS}28Z CAR-T cells after stimulation of CD19⁺ Raji cells was significantly lower than that of anti-CD19 28Z CAR-T cells in the later stage.

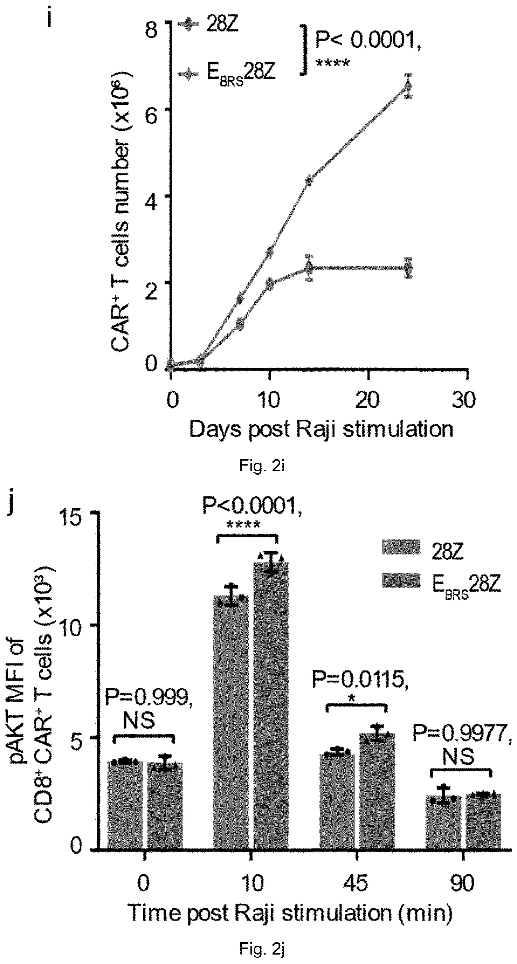

100,000 anti-CD19 28Z CAR-T cells and anti-CD19 E_{BRS}28Z CAR-T cells were respectively inoculated with CD19⁺ lymphoma cell line Raji in round-bottom 96-well plates at ratios of 1:1, then mixed well, afterward, were centrifuged (400 g, 1 min) at room temperature to promote cell-cell contact. The obtained samples were cultured with complete mediums putting in a 37°C incubator for a period of time. After sample collection at different time points, the collected samples were washed once with PBS, respectively, and the survival amount of CAR⁺T cells was detected with anti-human CD4-APC and anti-human CD8-PE-Cy7 antibody staining. As shown in Fig. 2i, the survival amount of anti-CD19 E_{BRS}28Z CAR-T cells after stimulation of CD19⁺ Raji cells was significantly higher than that of anti-CD19 28Z CAR-T cells.

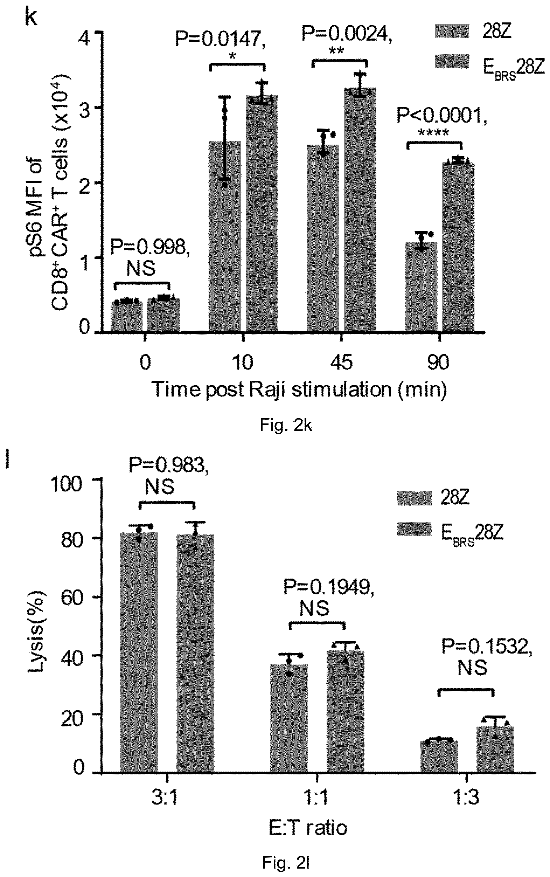

100,000 anti-CD19 28Z CAR-T cells and anti-CD19 E_{BRS}28Z CAR-T cells were respectively inoculated with CD19⁺ lymphoma cell line Raji at ratios of 1:1 in 1.5ml EP tubes, then mixed well, afterward, were centrifuged (500 g, 2 min) at 4°C to promote cell-cell contact. The obtained samples were cultured with complete mediums putting in a 37°C incubator for a period of time. 8% PFA with the same volumes were added at different time points for immobilization at room temperature for 5min, and then the samples were punched using 0.1% TritonX-100 at room temperature for 5min. Then, according to the intracellular staining known in the art, pAKT^{s473} rabbit antibody (Fig. 2j), and pS6^{S235/236} rabbit antibody (Fig. 2k) were first used as the primary antibodies to stain and washed once, latter goat anti-rabbit IgG labeled with Alexa647 were used as the second antibodies to stain and washed once, and afterward the flow cytometry was performed. As shown in Fig. 2j and Fig. 2k, the overall levels of pAKT^{S473} and pS6^{S235/236} of anti-CD19 E_{BRS}28Z CAR-T cells after antigen-specific stimulation were significantly higher than those of anti-CD19 28Z CAR-T cells. The phosphorylation level of the signaling molecule AKT^{S473} indicates the ability to promote cell proliferation and viability, and the phosphorylation level of the signaling molecular ribosomal protein S6^{S235/236} manifests the cell growth and proliferation level. These two indicators illustrate that the proliferation capability and viability of anti-CD19 E_{BRS}28Z CAR-T cells are stronger from the perspective of signaling pathway.

Cytotoxicity of anti-CD19 28Z CAR-T cells and E_{BRS}28Z CAR-T cells against CD19⁺ tumor cells. CD19⁻K562 cells were resuspended in a 1640 serum-free medium, then pre-stained with 1x CellTraker deep red dye (37 ° water bath, 30min), next were washed with 1640 serum-free medium once, and afterward were resuspended in complete T cell medium. The resuspended CD19⁻K562 cells were mixed with CD19⁺K562_ CD19 (IRES mCherry) cells at a ratio of 1:1 to serve as target cells. Anti-CD19 28Z CAR-T cells and anti-CD19 E_{BRS}28Z CAR-T cells were mixed with the above target cells in around-bottom 96-well plates at ratios of 3:1, 1:1, and 1:3, respectively, then mixed well, and afterward centrifuged (400g, 1min) at room temperature to promote cell-cell contact. The obtained samples were cultured with complete mediums putting in a 37°C incubator for one day. The samples were collected and placed in ice. The percentage of CD19⁻K562 cells (APC⁺, mCherry⁻) and the percentage of CD19⁺K562_CD19 cells (APC⁻, mCherry⁺) were detected by flow cytometry, where "the percentage of CD19⁻K562 cells (APC⁺, mCherry⁻)/the percentage of CD19⁺K562_CD19 cells (APC⁻, mCherry⁺)" in the experimental group was recorded as N. N was standardized with N₀, where "N₀= the percentage of CD19K562 cells (APC⁺, mCherrγ) / the percentage of CD19⁺K562_CD19 cells (APC⁻, mCherry⁺)" of the target cells in which no anti-CD19 28Z CAR-T cells or anti-CD19 E_{BRS}28Z CAR-T cells were added. The survival ratio was calculated as N/N₀ after standardization, and the killing rate formula was "Lysis(%)=1-N/N₀". As shown in Fig. 2l, the cytotoxicity of anti-CD19 E_{BRS}28Z CAR-T cells against CD19⁺ tumor cells was similar to that of anti-CD19 28Z CAR-T cells.

Experimental data processing methods are shown as follows.

The software GraphPad Prism 8.0.2 was used for data statistical analysis. When P<0.05, the difference was significant, specifically expressed as * P<0.05, ** P<0.01, *** P<0.001, **** P<0.0001, and each group of data was expressed in Mean ± SD.

Fig. 2d - Fig. 2f: one-way ANOVA with Sidak's multiple comparison.

Fig. 2g, Fig. 2h, Fig. 2j - Fig. 2l: two-way ANOVA with Sidak's multiple comparison.

Fig. 2i: two-way ANOVA.

Experimental results are shown as follows.
(1)The surface CAR levels and the CD4/CD8 ratios of the anti-CD19 E_{BRS}28Z CAR-T cells and anti-CD19 28Z CAR-T cells were similar.
(2) After anti-CD19 28Z CAR-T cells and anti-CD19 E_{BRS}28Z CAR-T cells were specifically stimulated by Raji cells in vitro, the amount of cytokines (IL-2, and TNF-α) secreted by the anti-CD19 EBRS28Z CAR-T cells was similar to that of the anti-CD19 28Z CAR-T cells, while the amount of cytokine IFN-γ generated from the anti-CD19 E_{BRS}28Z CAR-T cells was less than that of the anti-CD19 28Z CAR-T cells.
(3) After anti-CD19 28Z CAR-T cells and anti-CD19 E_{BRS}28Z CAR-T cells were specifically stimulated by Raji cells in vitro and amplified for a period of time, the proliferation capacities (namely, Ki67 expression level) were sampled and detected at different time points, and the proliferation capacity of the anti-CD19 E_{BRS}28Z CAR-T cell was significantly higher than that of the anti-CD19 28Z CAR-T cell.
(4) After anti-CD19 28Z CAR-T cells and anti-CD19 E_{BRS}28Z CAR-T cells were specifically stimulated by Raji cells in vitro and amplified for a period of time, the apoptosis levels were sampled and detected at different time points. The apoptosis levels of the anti-CD19 E_{BRS}28Z CAR-T cells and the anti-CD19 28Z CAR-T cells were similar in the early stage, but the apoptosis level of the anti-CD19 E_{BRS}28Z CAR-T cells was significantly lower than that of the anti-CD19 28Z CAR-T cells in the later stage.
(5) After anti-CD19 28Z CAR-T cells and anti-CD19 E_{BRS}28Z CAR-T cells were specifically stimulated by Raji cells and amplified for a period of time, the number of the amplified CAR⁺T cells was detected at different time points, and the number of the anti-CD19 E_{BRS}28Z CAR-T cells was significantly more than that of the anti-CD19 28Z CAR-T cells.
(6) After anti-CD19 28Z CAR-T cells and anti-CD19 E_{BRS}28Z CAR-T cells were specifically stimulated by Raji cells and amplified for a period of time, the phosphorylation levels of the signaling molecule AKT^{S473} were detected at different time points. The phosphorylation level of the signaling molecule AKT^{S473} indicates the ability to promote cell proliferation and viability. The overall level of pAKT^{S473} of anti-CD19 E_{BRS}28Z CAR-T cells was higher than that of anti-CD19 28Z CAR-T cells, indicating that the proliferation capability and viability of anti-CD19 E_{BRS}28Z CAR-T cells were higher than those of anti-CD19 28Z CAR-T cells.
(7) After anti-CD19 28Z CAR-T cells and anti-CD19 E_{BRS}28Z CAR-T cells were specifically stimulated by Raji cells and amplified for a period of time, the phosphorylation levels of the signaling molecule S6^{S235/236} were detected at different time points. The phosphorylation level of the signaling molecule S6^{S235/236} indicates cell growth and proliferation abilities. The overall level of pS6^{S235/236} of anti-CD19 E_{BRS}28Z CAR-T cells was significantly higher than that of anti-CD19 28Z CAR-T cells, indicating that the growth and proliferation abilities of anti-CD19 E_{BRS}28Z CAR-T cells were significantly higher than those of anti-CD19 28Z CAR-T cells.
**(8)** In the in vitro killing experiment, where anti-CD19 28Z CAR-T cells and anti-CD19 E_{BRS}28Z CAR-T cells were co-incubated with CD19⁺K562:CD19⁻K562, the killing ability of anti-CD19 E_{BRS}28Z CAR-T cells was similar to that of anti-CD19 28Z CAR-T cells.

For the comparison of in vivo anti-subcutaneous tumor ability between anti-CD19 E_{BRS}28Z CAR-T cells and anti-CD1928Z CAR-T cells, the experimental scheme is as follows:
**3×10^7/ml** Raji cell-PBS resuspension was prepared in aseptic conclusion, 100 ul (3 million) Raji cells were inoculated subcutaneously to the left back of each 6-week-old B-NDG female mouse, and the time of inoculation was denoted as day0. Six days later, the diameter of the Raji subcutaneous tumor became 7-8 mm. The mice then were randomly divided into three groups (E_{BRS}28Z group, 28Z group, and vector group), afterward, 100ul (8 million) T cells were injected into the tail vein of each mouse. After that, the size of the tumor was measured regularly with a vernier caliper, and the tumor data and survival period of the mice were recorded. The tumor area= the length x the width. The experimental conclusion is anti-CD19 E_{BRS}28Z CAR-T has an anti-tumor effect.

**The** above are merely preferred embodiments of the present disclosure, and are not limitations to the form and essence of the present disclosure. It should be noted that for a person of ordinary skill in the art, a number of improvements and supplements can be made without departing from the method of the present disclosure, and these improvements and supplements should also be regarded as the protection scope of the present disclosure. A person skilled in the art, without deviating from the spirit and scope of the present disclosure, can make some equivalent changes of alteration, modification, and variation according to the technical content disclosed above, which should be regarded as the equivalent embodiments of the present disclosure. And any equivalent changes, modifications, and variations to the above embodiments according to the essential technology of the present disclosure still fall within the scope of the technical solutions of the present disclosure.

## Claims

1. A chimeric antigen receptor containing a CD3ε intracellular region with a Y/F mutation, comprises:
an extracellular domain, a transmembrane domain, and an intracellular domain which are connected in sequence;
wherein the extracellular domain comprises an antigen recognition region and a hinge region; and
one end of the intracellular domain which is connected to the transmembrane domain is connected to a CD3ε intracellular region with a Y/F mutation, wherein two tyrosines in the immunoreceptor tyrosine-based activation motif of CD3ε intracellular region with a Y/F mutation are mutated into phenylalanine.

2. The chimeric antigen receptor containing a CD3ε intracellular region with a Y/F mutation according to claim 1, further comprises one or more of the following:
(1) the amino acid sequence of the CD3ε intracellular region with a Y/F mutation is shown as SEQ ID NO: 1;
(2) the intracellular domain comprises the CD3ε intracellular region with a Y/F mutation, a costimulatory signaling region, and a CD3ζ intracellular segment that are sequentially connected.

3. The chimeric antigen receptor containing a CD3ε intracellular region with a Y/F mutation according to claim 2, wherein the costimulatory signaling region is selected from one or more of intracellular segments of CD27, CD28, CD134, 4-1BB, OX40, and ICOS.

4. The chimeric antigen receptor containing a CD3ε intracellular region with a Y/F mutation according to claim 3, further comprises one or more of the following:
(1) the amino acid sequence of the CD28 intracellular segment is shown as SEQ ID NO: 2;
(2) the amino acid sequence of the CD3ζ intracellular segment is shown as SEQ ID NO: 3.

5. The chimeric antigen receptor containing a CD3ε intracellular region with a Y/F mutation according to claim 1, further comprises one or more of the following:
a. the antigen recognition region is selected from a single-chain antibody against a tumor surface antigen, and the tumor surface antigen is selected from one or more of CD19, mesothelin, CD20, CD22, CD123, CD30, CD33, CD38, CD138, BCMA, Fibroblast activation protein, Glypican-3, CEA, EGFRvIII, PSMA, Her2, IL13Rα2, CD171, and GD2;
b. the transmembrane domain is selected from one or more of transmembrane regions of CD28, CD4, CD8α, OX40, and H2-Kb;
c. the hinge region is selected from one or more of CD28 hinge region, CD8α hinge region, CD4 hinge region, IgG hinge region, or a coupled hinge region of IgG hinge region and CH2CH3 region.

6. The chimeric antigen receptor containing a CD3ε intracellular region with a Y/F mutation according to claim 5, further comprises one or more of the following:
d. the single-chain antibody is selected from FMC63;
e. the amino acid sequence of the CD28 transmembrane region is shown as SEQ ID NO: 4;
f. the amino acid sequence of the CD28 hinge region is shown as SEQ ID NO: 7.

7. The chimeric antigen receptor containing a CD3ε intracellular region with a Y/F mutation according to any one of claims 1-6, further comprises one or more of the following:
1) the antigen recognition region of the chimeric antigen receptor containing a CD3ε intracellular region with a Y/F mutation is selected from FMC63, the hinge region is selected from the CD28 hinge region, the transmembrane domain is selected from the CD28 transmembrane region, and the intracellular domain includes the CD3ε intracellular region with a Y/F mutation, the CD28 intracellular segment, and the CD3ζ intracellular segment that are sequentially connected;
2) the amino acid sequence of the chimeric antigen receptor containing a CD3ε intracellular region with a Y/F mutation is shown as SEQ ID NO: 5.

8. A polynucleotide sequence, wherein the polynucleotide sequence is selected from:
(1) a polynucleotide sequence which encodes the chimeric antigen receptor containing a CD3ε intracellular region with a Y/F mutation according to any one of claims 1-7;
(2) a complementary sequence to the polynucleotide sequence in (1).

9. The polynucleotide sequence according to claim 8, wherein the amino acid sequence of the polynucleotide sequence is shown as SEQ ID NO: 6.

10. A nucleic acid construct, comprises the polynucleotide sequence according to any one of claims 8-9,
preferably, the nucleic acid construct is a vector;
more preferably, the nucleic acid construct is a lentivirus vector, which contains a replication initiation site, a 3'LTR, a 5'LTR, and the polynucleotide sequence according to any one of claims 8-9.

11. A lentivirus vector system, comprises the nucleic acid construct according to claim 10 and a lentivirus vector auxiliary component.

12. A genetically modified T cell, comprises the polynucleotide sequence according to any one of claims 8-9 or the nucleic acid construct according to claim 10, or infects the lentivirus vector system according to claim 11.

13. A use of the chimeric antigen receptor containing a CD3ε intracellular region with a Y/F mutation according to any one of claims 1-7, the polynucleotide sequence according to any one of claims 8-9, the nucleic acid construct according to claim 10, or the lentivirus vector system according to claim 11 in any one or more of the following: (1) preparing T cells; (2) improving T cell viability; (3) inhibiting T cells from apoptosis; (4) enhancing T cell proliferation and/or viability; (5) improving the anti-tumor effect of T cells; (6) inhibiting T cells from secreting the cytokines IFN-γ and TNF-α.

14. A use of the chimeric antigen receptor containing a CD3ε intracellular region with a Y/F mutation according to any one of claims 1-7, the polynucleotide sequence according to any one of claims 8-9, the nucleic acid construct according to claim 10, the lentivirus vector system according to claim 11, or the genetically modified T cell according to claim 12 in the preparation of tumor treatment products.

15. A chimeric antigen receptor containing a CD3ε intracellular basic residue rich sequence, comprises:
an extracellular domain, a transmembrane domain, and an intracellular domain which are connected in sequence;
wherein the extracellular domain comprises an antigen recognition region and a hinge region; and
one end of the intracellular domain which is connected to the transmembrane domain is connected to a CD3ε intracellular basic residue rich sequence.

16. The chimeric antigen receptor containing a CD3ε intracellular basic residue rich sequence according to claim 15, further comprises one or more of the following:
(1) the amino acid sequence of the CD3ε intracellular basic residue rich sequence is shown as SEQ ID NO: 10;
(2) the intracellular domain comprises the CD3ε intracellular basic residue rich sequence, a costimulatory signaling region, and a CD3ζ intracellular segment that are sequentially connected.

17. The chimeric antigen receptor containing a CD3ε intracellular basic residue rich sequence according to claim 16, wherein the costimulatory signaling region is selected from one or more of intracellular segments of CD27, CD28, CD134, 4-1BB, OX-40, and ICOS.

18. The chimeric antigen receptor containing a CD3ε intracellular basic residue rich sequence according to claim 17, further comprises one or more of the following:
(1) the amino acid sequence of the CD28 intracellular segment is shown as SEQ ID NO: 2;
(2) the amino acid sequence of the CD3ζ intracellular segment is shown as SEQ ID NO: 3.

19. The chimeric antigen receptor containing a CD3ε intracellular basic residue rich sequence according to claim 15, further comprises one or more of the following:
a. the antigen recognition region is selected from a single-chain antibody against a tumor surface antigen, and the tumor surface antigen is selected from one or more of CD19, mesothelin, CD20, CD22, CD123, CD30, CD33, CD38, CD138, BCMA, Fibroblast activation protein, Glypican-3, CEA, EGFRvIII, PSMA, Her2, IL13Rα2, CD171, and GD2;
b. the transmembrane domain is selected from one or more of transmembrane regions of CD28, CD4, CD8α, OX40, and H2-Kb;
c. the hinge region is selected from one or more of CD28 hinge region, CD8α hinge region, CD4 hinge region, IgG hinge region, or a coupled hinge region of IgG hinge region and CH2CH3 region.

20. The chimeric antigen receptor containing a CD3ε intracellular basic residue rich sequence according to claim 19, further comprises one or more of the following:
d. the single-chain antibody is selected from FMC63;
e. the amino acid sequence of the CD28 transmembrane region is shown as SEQ ID NO: 4;
f. the amino acid structure of the CD28 hinge region is shown as SEQ ID NO: 7.

21. The chimeric antigen receptor containing a CD3ε intracellular basic residue rich sequence according to any one of claims 15-20, further comprises one or more of the following:
1) the antigen recognition region of the chimeric antigen receptor containing a CD3ε intracellular basic residue rich sequence is selected from FMC63, the hinge region is selected from CD28 hinge region, the transmembrane domain is selected from CD28 transmembrane region, and the intracellular domain includes the CD3ε intracellular basic residue rich sequence, the CD28 intracellular segment, and the CD3ζ intracellular segment that are sequentially connected;
2) the amino acid sequence of the chimeric antigen receptor containing a CD3ε intracellular basic residue rich sequence is shown as SEQ ID NO: 11.

22. A polynucleotide sequence, wherein the polynucleotide sequence is selected from:
(1) a polynucleotide sequence which encodes the chimeric antigen receptor containing a CD3ε intracellular basic residue rich sequence according to any one of claims 15-21;
(2) a complementary sequence to the polynucleotide sequence in (1).

23. The polynucleotide sequence according to claim 22, wherein the polynucleotide sequence is shown as SEQ ID NO: 12.

24. A nucleic acid construct, comprises the polynucleotide sequence according to any one of claims 22-23;
preferably, the nucleic acid construct is a vector;
more preferably, the nucleic acid construct is a lentivirus vector, which contains a replication initiation site, a 3'LTR, a 5'LTR, and the polynucleotide sequence according to any one of claims 22-23.

25. A lentivirus vector system, comprises the nucleic acid construct according to claim 24 and a lentivirus vector auxiliary component.

26. A genetically modified T cell, comprises the polynucleotide sequence according to any one of claims 22-23 or the nucleic acid construct according to claim 24, or infects the lentivirus vector system according to claim 25.

27. A use of the chimeric antigen receptor containing a CD3ε intracellular basic residue rich sequence according to any one of claims 15-21, the polynucleotide sequence according to any one of claims 22-23, the nucleic acid construct according to claim 24, or the lentivirus vector system according to claim 25 in any one or more of the following: (1) preparing T cells; (2) improving T cell viability; (3) inhibiting T cells from apoptosis; (4) enhancing T cell proliferation; (5) improving the anti-tumor effect of T cells; (6) inhibiting T cells from secreting the cytokine IFN-γ.

28. A use of the chimeric antigen receptor containing a CD3ε intracellular basic residue rich sequence according to any one of claims 15-21, the polynucleotide sequence according to any one of claims 22-23, the nucleic acid construct according to claim 24, the lentivirus vector system according to claim 25, or the genetically modified T cell according to claim 26 in preparation of tumor treatment products.
